# EUROPEAN PATENT SPECIFICATION

(11) **EP 1 141 334 B1**
(45) Date of publication and mention of the grant of the patent: **08.10.2008**
(21) Application number: 99963641.8
(22) Date of filing: 23.12.1999
(51) Int. Cl.: C12N 15/55

(54) **HUMAN GLYCOSYLPHOSPHATIDYLINOSITOL SPECIFIC PHOSPHOLIPASE D VARIANTS AND USES THEREOF**
VARIANTEN DER HUMANEN GLYCOSYLPHOSPHATIDYLINOSITOL-SPEZIFISCHEN PHOSPHOLIPASE D UND IHRE VERWENDUNGEN
PROTEINES D PHOSPHOLIPASE SPECIFIQUES DU GLYCOSYL PHOSPHATIDY LINOSITOL ET LEURS UTILISATIONS

(30) Priority: 24.12.1998 GB 9828712; 24.12.1998 GB 9828715; 24.12.1998 GB 9828713
(43) Date of publication of application: 10.10.2001
(73) Proprietor: Sylus Pharmaceuticals Ltd, London, EC4A 3LX (GB)
(72) Inventor: SCHOFIELD, Julian, London EC1V 7JA (GB); RADEMACHER, Thomas William, Boars Hill, Oxford OX1 5EY (GB)
(74) Representative: Kiddle, Simon John
(86) International application number: PCT/GB1999/004399
(87) International publication number: WO 2000/039285

(56) References cited:
- EP-A- 0 477 739
- WO-A-99/47565
- MAGUIRE, G.A. & GOSSNER, A.: "Glycosyl phosphatidyl inositol phospholipase D activity in human serum" ANNALS OF CLINICAL BIOCHEMISTRY, vol. 32, no. 1, January 1995 (1995-01), pages 74-78, XP000864653
- VICENT, D. ET AL.: "Alterations in Skeletal Muscle Gene Expression" DIABETES, vol. 47, no. 9, September 1998 (1998-09), pages 1451-1458, XP000864657
- SCALLON, B.J. ET AL.: "Primary Structure and Fucntional Acitvity of a Phosphatidylinositol-Glycan-Specific Phospholipase D" SCIENCE, vol. 252, no. 5004, 19 April 1991 (1991-04-19), pages 446-448, XP000293055 cited in the application
- TSANG, T.C. ET AL.: "Isolation and expression of two human glycosylphosphatidylinositol phospholipase D (GPI-PLD) cDNAs" FASEB JOURNAL, vol. 6, April 1992 (1992-04), page A1922 XP000907489 cited in the application -& EMBL Database, Heidelberg, FRG accession number L11702 07 September 1993 TSANG, T.C. ET AL: "Human phospholipase D mRNA, complete cds" XP002141248 cited in the application
- HOENER, M.C. & BRODBECK, U.: "Phosphatidylinositol-glycan-specific phospholipase D is an amphiphilic glycoprotein that in serum is associated with high-density lipoproteins" EUROPEAN JOURNAL OF BIOCHEMISTRY, vol. 206, no. 3, June 1992 (1992-06), pages 747-757, XP000913778 cited in the application
- HUANG, L.C ET AL.: "Chiroinositol Deficiency and Insulin Resistence. III. Acute Glycogenic and Hypoglycemic Effects of Two Inositol Phosphoglycan Insulin Mediators in Normal and Streptozotocin-Diabetic Rats in Vivo" ENDOCRINOLOGY, vol. 132, no. 2, January 1993 (1993-01), pages 652-657, XP002050432

## Description

### Field of the Invention

The present invention relates to glycosylphosphatidylinositol specific phospholipase D (GPI-PLD) proteins and uses of these proteins, in particular in the treatment diabetes and complications of diabetes such as insulin resistance, liver dysfunction, disorders involving pancreatectomies and conditions mediated by a product of an infectious organism which is capable of inhibiting GPI-PLD, such as septic shock. The present invention further relates to variant GPI-PLD polypeptides.

### Background of the Invention

Studies have shown that a number of cell surface proteins are attached to the cell membrane by covalent linkage to a glycosylphosphatidylinositol (GPI) anchor. It has been shown that the enzyme GPI-PLD cleaves the phosphodiester bond linking glycosylphosphatidylinositol to phosphatidic acid, thereby releasing anchored proteins.

GPI-PLD enzymes are abundantly present in human and bovine serum (5-10mg/ml in human serum). US Patent No: 5,418,147 and EP 0 477 739 A (Huang et al) describes the purification of GPI-PLD from bovine liver, and the subsequent cloning of three GPI-PLD enzymes from bovine liver, human liver and human pancreas cDNA libraries. This patent reports the full length cDNA and amino acid sequences of the GPI-PLDs from human and bovine liver, and the partial cDNA and amino acid sequences of the human pancreatic form of the enzyme. Subsequently, the full length sequence of the pancreatic form of GPI-PLD was reported in Tsang et al (1992), and this enzyme has been found in cDNA libraries from breast, eye, spleen and tonsil. The three forms of the enzymes are highly homologous with the predicted mature protein sequences of bovine liver GPI-PLD sharing 82% sequence identity with the human liver enzyme and 81% sequence identity with the human pancreatic enzyme. The amino acid sequences of human liver and pancreatic forms of GPI-PLD were deposited at GenBank under accession numbers L11701 and L11702 and consist of 841 and 840 amino acids respectively. The human liver and pancreatic forms of GPI-PLD share 94.6% sequence identity. The structure of GPI-PLDs is further discussed in Scallon et al, 1991.

However, despite cloning three forms of GPI-PLD, there is no suggestion in these references as to the *in vivo* role of the enzymes. Further, the only application of the enzymes suggested is in an expression system in which a heterologous protein is expressed in a host cell as a fusion with a GPI-signal peptide, leading to the heterologous protein becoming anchored to the cell membrane by a GPI anchor, where it can be cleaved off by coexpressed or added GPI-PLD.

GPI-PLD has also been isolated from human serum by Hoener et al (1992) and this form of the enzyme was found to be identical to the human pancreatic GPI-PLD apart from changes at 531 to 534 where VIGS is replaced by MLGT. This paper also showed that treatment of serum GPI-PLD with N-glycosidase F reduced the apparent molecular weight from 123 kD to 87 kD. Similarly, Li et al (1994) have shown that GPI-PLD was cleaved by trypsin into 3 fragments (2 x 40 kD and 30 kD), and Heller et al (1994) have shown that 33, 39 and 47kD species were produced, with only the N-terminal 39 kD fragment moiety showing enzyme activity after renaturation.

It has been proposed that one function of GPI-PLD enzyme is to produce inositolphosphoglycans (IPGs) by the cleavage of "free" GPIs in the plasma membrane in response to binding of a growth factor to its receptor (Rademacher et al, 1994). This role for GPI-PLD has been demonstrated in mast cells where IgE-dependent activation of these cells results in release of their granule contents, which include substances such as histamine, a mediator of the inflammatory response. In the presence of antigen, histamine is released; this release can be mimicked by addition of IPGs and is blocked by addition of anti-GPI-PLD antibodies (Lin et al, 1991).

Maguire and Gossner (Annals of Chemical Biochemistry, 32(1): 74-78, 1995) describe the measurement of GPI-PLD activity in human serum. Patients with liver disease had lower activities than control, while patients with renal disease were higher. The authors note that the function of GPI-PLD is unknown. Huang et al (Endocrinology, 132(2): 652-657, 1993) describes the purification of insulin mediators from rats.

The role of GPI-PLD in cleaving GPI-anchored proteins, and especially inositolphosphoglycans (IPGs), is examined in Jones et al (1997). However, the authors reflect the uncertainty in the art regarding the mechanism of IPG generation, noting that "The definitive activated enzyme, being a GPI-PLC or a GPI-PLD, has yet to be unequivocally identified" and that "little attention has been payed to the role of GPI-PLD as the hydrolysing enzyme".

In summary, despite the cloning of GPI-PLD enzymes and investigation as to their biochemical properties, the role of the enzyme in vivo or any possible medical use remains unknown.

### Summary of the Invention

Broadly, the present invention relates to GPI-PLD for medical use, and in particular for the treatment of conditions which respond to GPI-PLD or which are characterised by reduced levels of active GPI-PLD in patients. The present invention relates in particular to the use of GPI-PLD in the treatment and diagnosis of diabetes and complications of diabetes, liver dysfunction and disorders involving pancreatectomies. The GPI-PLD can be the forms of the enzyme disclosed in the prior art, or the GPI-PLDs disclosed for the first time here, including GPI-PLD variants which have a reduced susceptibility to phosphorylation by cAMP dependent protein kinase (PKA).

Accordingly, in first aspect, the present invention provides GPI-PLD for use in a method of therapy as set ont in claim 1.

In a further aspect, the present invention provides a nucleic acid molecule encoding GPI-PLD.for use in a method of therapy as set out un claim 8.

In a further aspect, the present invention provides the use of glycosylphosphatidylinositol specific phospholipase D (GPI-PLD) for the preparation of a medicament for the treatment of conditions that respond to GPI-PLD cr which are characterised by reduced levels of active GPI-PLD as compared to a normal patient.

In a first embodiment, the present invention relates to the role of GPI-PLD in diabetes and diabetic complications.

Insulin is a major anabolic hormone and has both mitogenic and metabolic effects. Whilst much effort has been directed towards study of the cascade of intracellular phosphorylation events initiated by the binding of insulin to its cell surface receptor, the signalling arm mediated by IPGs has been largely overlooked. In one aspect, the present invention is based on the realisation that GPI-PLDs are in fact the enzymes responsible for production of IPG second messengers following the binding of insulin to its receptor. The IPGs then interact with other cellular enzymes instigating some of the metabolic effects of the hormone. In particular, diabetic complications such as insulin resistance may be caused by deficiencies in GPI-PLD. Pancreatic islet cells produce and secrete GPI-PLD, which is transported in blood complexed with apolipoprotein A1, and may therefore represent the major source of circulating enzyme.

Insulin resistance is seen in both the early stages of type I (IDDM) and type II diabetes mellitus (NIDDM). If GPI-PLD levels are depleted by the destruction of pancreatic b-cells, as is seen in streptozotocin-treated rats, then this could be an important factor in the development of insulin resistance. This in turn suggests the treatment of such patients with GPI-PLD, optionally in combination with other diabetes therapies.

In a further aspect, the present invention provides the use of GPI-PLD for the preparation of a medicament for the treatment of diabetes, and in particular insulin dependent forms of diabetes.

In a further aspect, the present invention provides the use of GPI-PLD for the preparation of a medicament for the treatment of complications of diabetes, and in particular the treatment of insulin resistance. effective amount of GPI-PLD.

In all of the above aspects, GPI-PLD can be administered alone or in conjunction with other treatments for diabetes or diabetic complications, either sequentially or simultaneously.

This therapeutic use of GPI-PLD may involve determining GPI-PLD levels or the levels of a product of GPI-PLD action, for example IPG or acyl-IPG, allowing the treatment of the patient to be tailored accordingly to the patient's individual needs.

In a second embodiment, the present invention relates to role of GPI-PLD in liver dysfunction and conditions involving pancreatectomies.

Thus, in a further aspect, the present invention provides the use of GPI-PLD for the preparation of a medicament for the treatment of liver dysfunction. Preferably, the GPI-PLD is administered in combination with apolipoprotein A1.

Treatment with GPI-PLD may also be applicable for patients with pancreatectomies and disorders associated with this state, in which case it is preferably administered with apolipoprotein A1 or another suitable carrier such as a liposome.

In all of the above aspects, GPI-PLD can be administered alone or in conjunction with other treatments for liver dysfunction, either sequentially or simultaneously.

In a further aspect, the present invention provides a pharmaceutical composition comprising a nucleic acid molecule encoding a GPI-PLD protein and apolipoprotein A1.

In a further embodiment, the present invention relates to the role of GPI-PLD in conditions mediated by a product of an infectious organism, such as septic shock.

The present invention is suitable for use in the treatment of conditions mediated by a product of an infectious organism which is capable of inhibiting GPI-PLD. The GPI-PLD can be of the forms of the enzyme disclosed in the prior art, or the GPI-PLDs disclosed for the first time here. An example of such a condition includes septic shock which commonly occurs following abdominal surgery, severe burns, trauma or cardiac failure. Septic shock is generally preceded by a reduction in splanchnic blood flow, resulting in ischaemia and epithelial damage on reperfusion, allowing ingress of microorganisms and subsequent sepsis. It is observed in conditions such as septic shock, endotoxin is released from the microorganisms causing sepsis, leading to the clinical symptoms of septic shock such as total organ failure and fatal shock. The endotoxins can be glycolipids released from gram negative bacteria or glycolipids such as LAM released from *Mycobacteria* such as *Tuberculosis*. Without wishing to be bound by any particular theory, these endotoxins are believed to act by inhibiting GPI-PLD.

At present, despite many attempts in the art to develop a treatment for septic shock and other related conditions, there are no approved treatments available. In particular, a reliable diagnostic test for determining whether a patient has or is at risk of developing conditions such as septic shock would be useful as an early warning of the condition and to allow timely treatment to be given.

In the above uses, the product of the infectious organism is typically an endotoxin, such as the glycolipids produced by gram negative or mycobacteria mentioned above.

This therapeutic use of GPI-PLD may involve determining the presence or amount of GPI-PLD or a product of GPI-PLD action in a biological sample from a patient. This determination can help in the diagnosis or prognosis of the patient, allowing the treatment of the patient to be tailored accordingly to the patient's individual needs. IPGs or the acyl IPGs produced by GPI-PLD action can be used in this diagnosis as the inhibition of GPI-PLD by endotoxins is likely to cause the level of IPGs (e.g. in urine, blood etc) to drop since the GPI-PLD causes the release of IPG precursors. Thus, monitoring either or both of the level of GPI-PLD or the IPGs provides a way of assessing the likelihood of developing conditions such as septic shock or their prognosis. A determination of the amount of GPI-PLD can be carried out using immobilised binding agents or by determining one or more of the activities associated with GPI-PLD and/or IPGs (see further below).

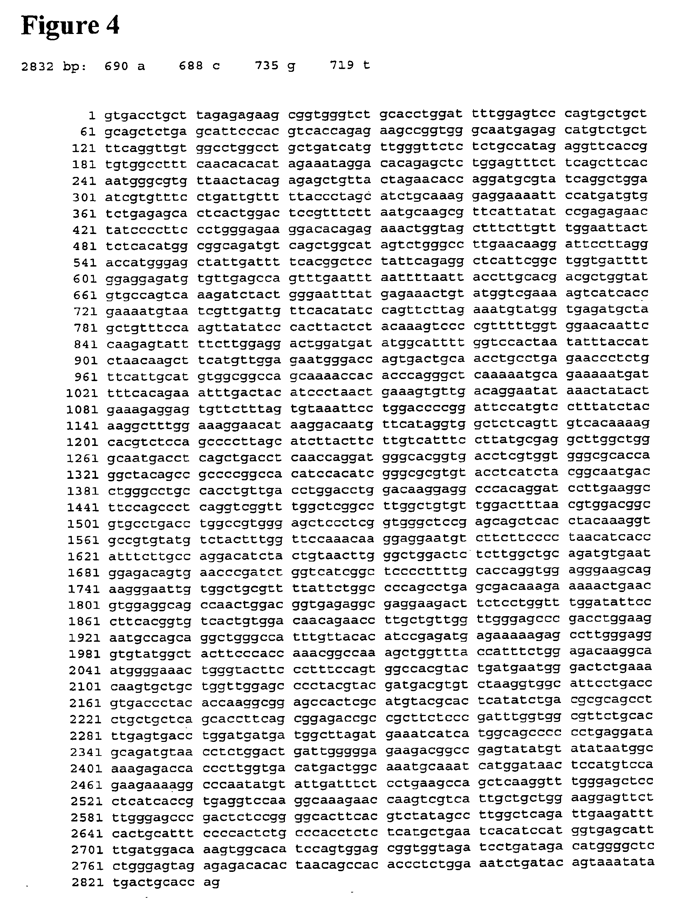

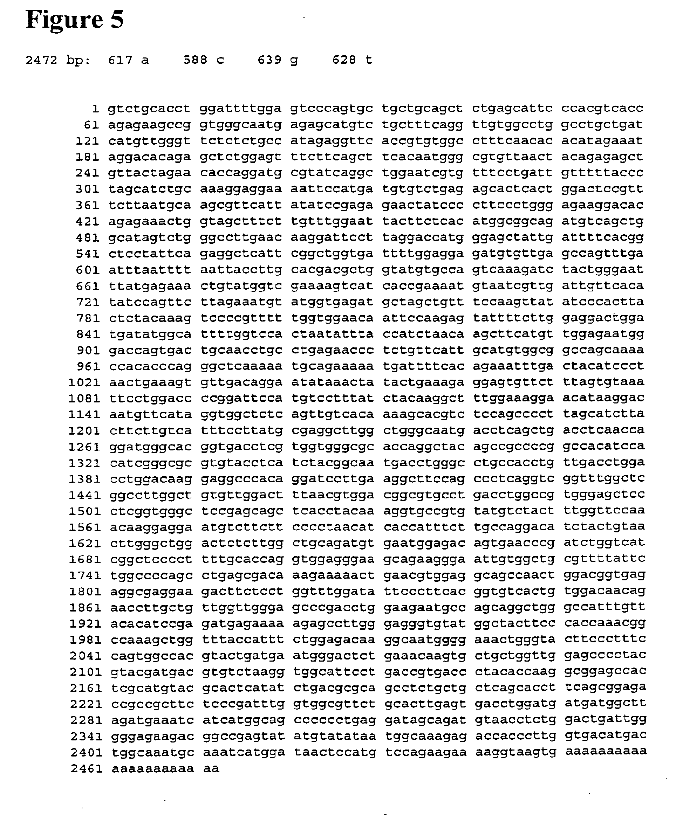

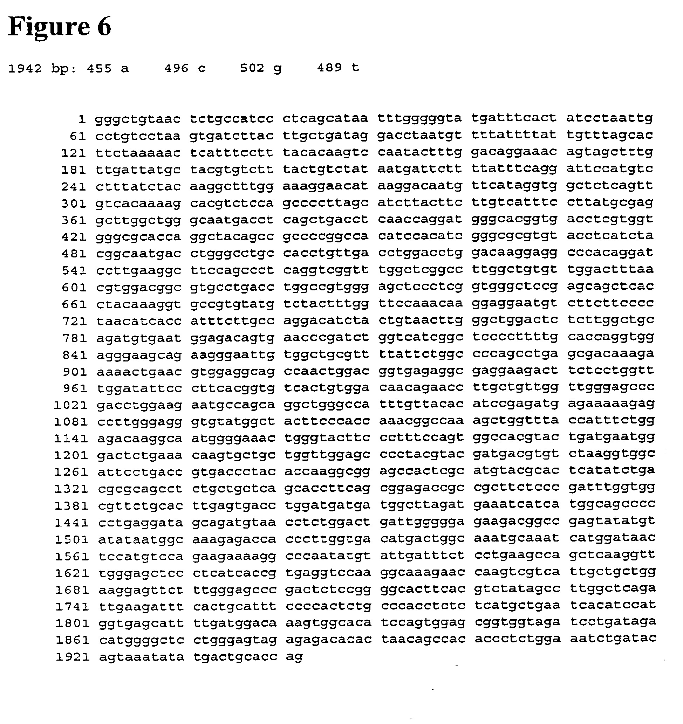

In a further general aspect, the present-invention provides an expression vector comprising nucleic acid encoding GPI-PLD for use in a method of gene therapy, e.g. in the treatment of patients unable to produce sufficient GPI-PLD. The GPI-PLD encoding nucleic acid can be a sequence shown in Figures 4 to 6 or one of the known nucleic acid sequences.

In a further general aspect, the present invention provides a cell line for transplantation into a patient, wherein the cell line is transformed with nucleic acid encoding GPI-PLD, and is capable of expressing and secreting GPI-PLD. In one embodiment, the cell line is encapsulated, e.g. in a biocompatible polymer, so that the GPI-PLD produced by the cell line can be secreted into the patient, while preventing rejection by the immune system of the host. Methods for encapsulating cells in biocompatible polymers are described in WO93/16687 and WO96/31199.

In a further general aspect, the present invention provides a pharmaceutical composition comprising a nucleic acid molecule encoding a GPI-PLD protein.

In a further aspect, the present invention provides a pharmaceutical composition comprising a GPI-PLD protein.

The present invention also relates to novel GPI-PLD proteins and nucleic acid molecules, and in particular to forms of the protein having sequence differences compared to the known human liver and pancreatic forms reported in the prior art.

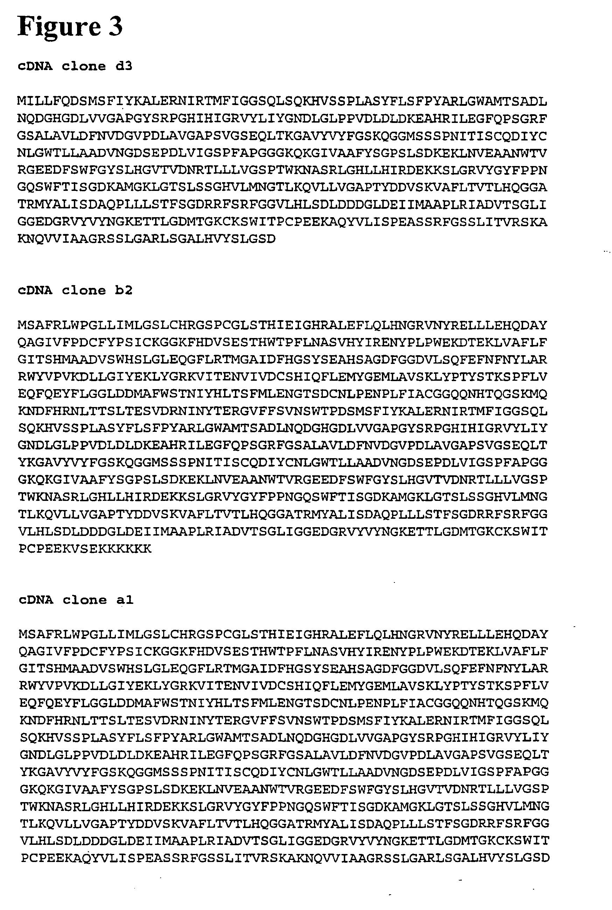

In a further aspect, the present invention provides a substance which is an isolated polypeptide comprising a polypeptide having the amino acid sequence set out in Figure 3.

In a further aspect, the present invention provides isolated nucleic acid molecules encoding any one of the above polypeptides. Examples of such nucleic acid sequences are the nucleic acid sequences set out in Figures 4 to 6. The present invention also includes nucleic molecules having, for example, greater than 90% sequence identity with the nucleic acid sequences shown in these figures.

In further aspects, the present invention provides an expression vector comprising the above GPI-PLD proteins, nucleic acid operably linked to control sequences to direct its expression, and host cells transformed with the vectors. The present invention also includes a method of producing the above GPI-PLD proteins comprising culturing the host cells and isolating the GPI-PLD thus produced.

We have now also identified a phosphorylation site on GPI-PLD acted on by cAMP protein dependent kinase (PKA) which switches off the activity of the enzyme. This in turn makes it possible to make GPI-PLD variants having a reduced tendency to be phosphorylated, and consequently have an improved activity profile, and utility in vitro or in vivo.

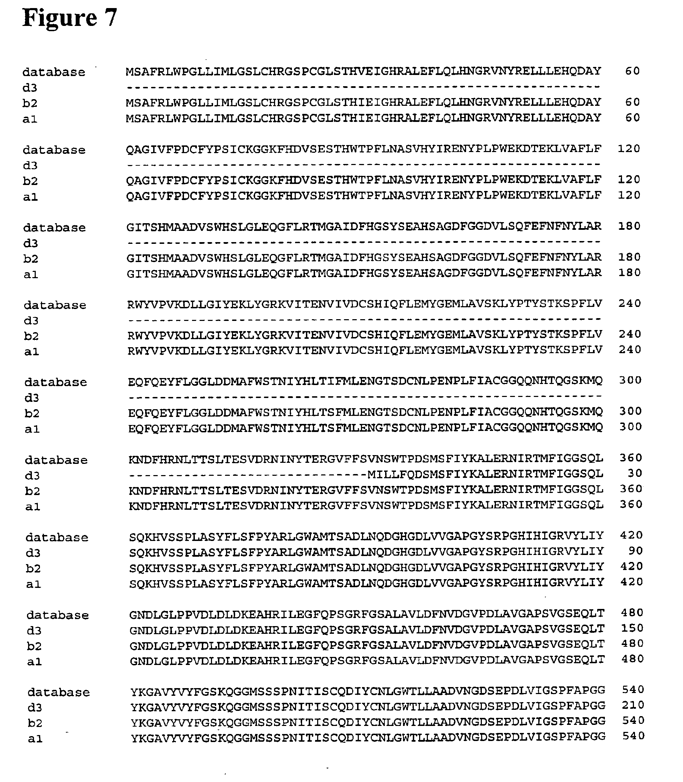

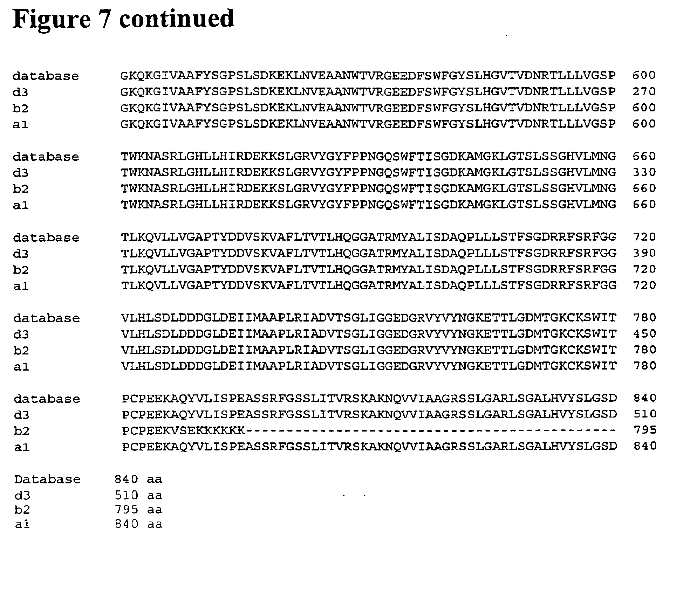

Accordingly, the present invention can employ variant GPI-PLD polypeptides differing in amino acid sequence in the region corresponding to amino acid residues 689-692 (RRFS) of mature human wild-type GPI-PLD (corresponding to residues 713-716 of the sequence shown in Figure 7). These proteins have a reduced tendency or cannot be phosphorylated by the PKA (which is itself activated by the A-type IPGs released by GPI-PLD), and so are likely to have increased activity or half-life when used *in vitro* or *in vivo*.

Thus, present invention identifies for the first time a region between amino acids 689-692 which when modified, e.g. by a substitution, deletion or insertion of one or more amino acids, disrupts the phosphorylation site in this region. Preferred modifications are substitutions, and in particular substitutions to change the serine residue at position 692 to an amino acid other than serine or threonine.

These and other aspects of the present invention are described in more detail below.

By way of example, embodiments of the present invention will now be described in more detail with reference to the accompanying figures.

### Brief Description of the Figures

Figure 1 shows an alignment of the deduced amino acid sequences of GPI-PLD encoded by cDNA clone A1 and the bovine and human liver GPI-PLD sequences disclosed in US Patent No: 5,418,147 (Huang et al).
Figure 2 shows the nucleic acid sequence from cDNA clone A1 aligned with the pancreatic forms of GPI-PLD disclosed in US Patent No: 5,418,147 (Huang et al) (partial sequence) and the corresponding full length nucleic acid sequence deposited at GenBank.
Figure 3 shows the amino acid sequences of the GPI-PLDs in clones al, b2 and d3, and consist of 840, 795 and 510 amino acids respectively.
Figure 4 shows the nucleic acid sequence of cDNA clone a1 encoding GPI-PLD, consisting of 2832 bp.
Figure 5 shows the nucleic acid sequence of cDNA clone b2 encoding GPI-PLD, consisting of 2472 bp.
Figure 6 shows the nucleic acid sequence of cDNA clone d3 encoding GPI-PLD, consisting of 1942 bp.
Figure 7 shows an alignment of the deduced amino acid sequences of GPI-PLDs encoded by cDNA clones a1, b2 and d3 with the pancreatic form of the enzyme deposited at GenBank under accession number 11702.
Figure 8 shows an alignment of the nucleic acid sequences from cDNA clones a1, b2 and d3 with the cDNA sequence encoding the human pancreatic form of GPI-PLD deposited at GenBank under accession number L11702.

### Detailed Description

### GPI-PLD Proteins

The term "GPI-PLD biological activity" is herein defined as the enzymatic activity of GPI-PLD in cleaving the photodiester bond linking glycosylphosphatidylinositol to phosphatidic acid, e.g. releasing a GPI-anchored protein. As noted in Heller et al (1994), this activity has been localised to the N-terminal 39 kD portion of full length GPI-PLD.

The medical uses of GPI-PLD described herein can use the novel GPI-PLD variants or the forms of the enzyme disclosed in the prior art. In either event, the skilled person can use the techniques described herein and others well known in the art to produce large amounts of these proteins, or fragments or active portions thereof, for use as pharmaceuticals, in the developments of drugs and for further study into its properties and role *in vivo*.

In a further aspect of the present invention provides a polypeptide having the amino acid sequence shown in Figure 3, which may be in isolated and/or purified form, free or substantially free of material with which it is naturally associated. In one embodiment, the clone a1 has an amino acid sequence consisting of 840 amino acids, a 23 amino acid signal peptide and a 817 amino acid mature protein. The present invention relates to both GPI-PLD proteins (and variants thereof) with and without the signal peptide, i.e. comprising amino acids 1-840 or 24-840 as shown in the figures.

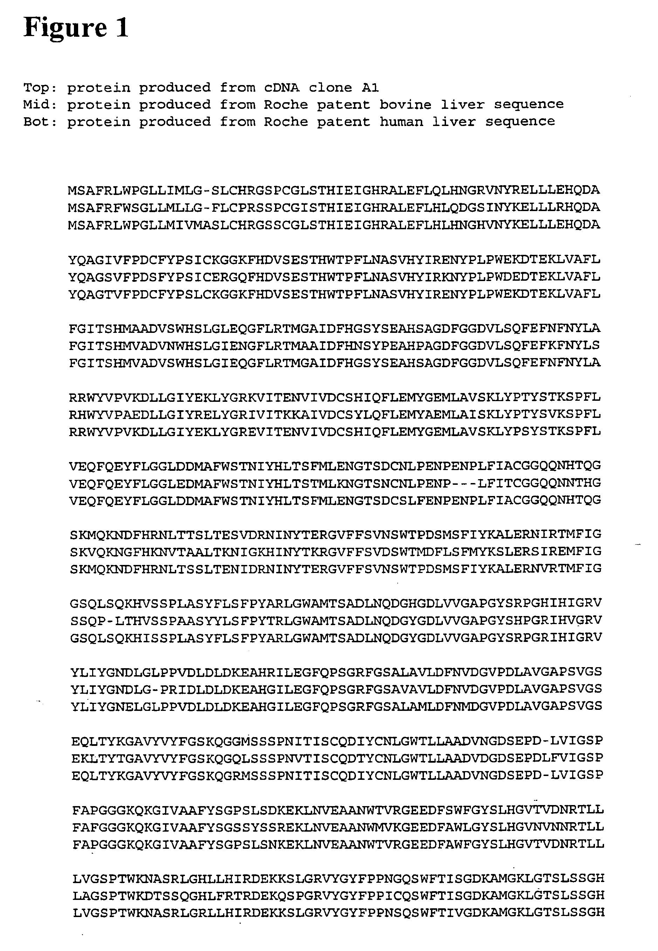

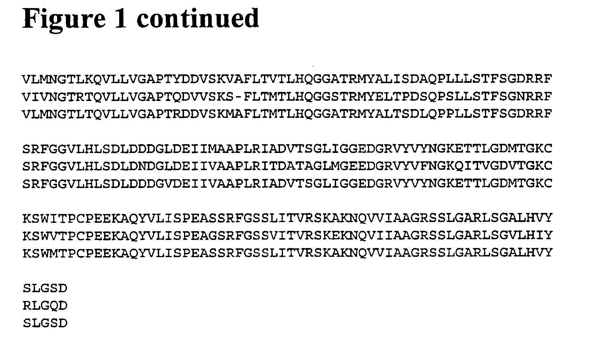

GPI-PLD proteins which are amino acid sequence variants or alleles can also be used in the present invention. A polypeptide which is a variant or allele may have an amino acid sequence which differs from that given in Figures 1 or 3 by one or more of addition, substitution, deletion and insertion of one or more amino acids. Preferred polypeptides have GPI-PLD enzymatic function as defined above.

A GPI-PLD protein which is an amino acid sequence variant or allele of an amino acid sequence shown in Figures 1 or 3 may comprise an amino acid sequence which shares greater than about 70%, greater than about 80%, greater than about 90%, greater than about 95%, greater than about 97%, greater than about 98% or greater than about 99% sequence identity with an amino acid sequence shown in Figures 1 or 3. Sequence comparison and identity calculations were carried out using the Cluster program (Thompson et al, 1994), using the following parameters (Pairwise Alignment Parameters: Weight Matrix: pam series; Gap Open Penalty: 10.00; Gap Extension Penalty: 0.10). Alternatively, the GCG program could be used which is available from Genetics Computer Group, Oxford Molecular Group, Madison, Wisconsin, USA, Version 9.1. Particular amino acid sequence variants may differ from those shown in Figures 1 and 3 by insertion, addition, substitution or deletion of 1 amino acid, 2, 3, 4, 5-10, 10-20 20-30, 30-50, 50-100, 100-150, or more than 150 amino acids.

The variant GPI-PLD polypeptides of the present invention differ in amino acid sequence as compared to human GPI-PLD at the phosphorylation site from amino acids 689 to 692 of the mature sequence (corresponding to amino acids 713-716 shown in Figure 7), i.e. within the amino acid motif RRFS. The term 'variant GPI-PLD polypeptide' is intended, inter alia, to include polypeptides which are modified within this region by deletion, substitution and/or insertion of one or more amino acids. These sequence differences may be the result of varying the GPI-PLD amino acid sequence of a parent GPI-PLD polypeptide, either a wild type GPI-PLD polypeptide or a GPI-PLD polypeptide comprising one or more other modifications, e.g. by manipulation of the nucleic acid encoding the polypeptide, by altering the polypeptide itself or by the de *novo* synthesis of the variant protein. In preferred embodiments, the GPI-PLD retains, at least in part, one of its biological activities, e.g. by the presence of a functional N-terminal domain.

A deletion may take the form of the deletion of one, two, three or all four amino acids within the region. In some embodiments, the deletion may be part of a larger deletion encompassing a greater part of the GPI-PLD molecule. In a preferred embodiment, the variant GPI-PLD polypeptides have an amino acid sequence which differs from the amino acid sequence of human wild type GPI-PLD by the deletion comprising residues 689 to 692 inclusive.

An insertion may take the form of 1, 2, 3, 4 or 5 or more additional amino acids inserted between amino acids within the RRFS motif to disrupt it.

A substitution may take the form of the substitution of one, two, three or all of the four amino acids within the region corresponding to amino acids 689 to 692 of wild type human GPI-PLD. The substitutions within this region may be part of a more extensive series of substitutions encompassing other parts of the GPI-PLD polypeptide. In particular, mutant forms of GPI-PLD which may have practical use differ from the wild type sequence. Some of these mutants are used in the experiments described below.

In all cases, it is preferred that the resulting GPI-PLD variant retains or has an increased GPI-PLD biological activity as compared to human wild type GPI-PLD, and more especially the enzymatic activity of GPI-PLD in cleaving the phosphodiester bond linking GPI to phosphatidic acid, and thereby releasing a GPI-anchored protein.

The present invention also includes the use of active portions and fragments of the GPI-PLD proteins.

An "active portion" of GPI-PLD protein is a polypeptide which is less than said full length GPI-PLD protein, but which retains at least one its essential biological activity, e.g. the enzyme activity mentioned above known to be located in the N-terminal 39kD portion of the enzyme. For instance, portions of GPI-PLD protein can act as sequestrators or competitive antagonists by interacting with other proteins.

A "fragment" of the GPI-PLD protein means a stretch of amino acid residues of at least about 5 to 7 contiguous amino acids, often at least about 7 to 9 contiguous amino acids, typically at least about 9 to 13 contiguous amino acids, more preferably at least about 20 to 30 or more contiguous amino acids, more preferably greater than 40 amino acids, more preferably greater than 100 amino acids.

A polypeptide according to the present invention may be isolated and/or purified (e.g. using an antibody) for instance after production by expression from encoding nucleic acid (for which see below). Polypeptides according to the present invention may also be generated wholly or partly by chemical synthesis. The isolated and/or purified polypeptide may be used in formulation of a composition, which may include at least one additional component, for example a pharmaceutical composition including a pharmaceutically acceptable excipient, vehicle or carrier. A composition including a polypeptide according to the invention may be used in prophylactic and/or therapeutic treatment as discussed below.

The GPI-PLD polypeptides can also be linked to a coupling partner, e.g. an effector molecule, a label, a drug, a toxin and/or a carrier or transport molecule. Techniques for coupling the peptides of the invention to both peptidyl and non-peptidyl coupling partners are well known in the art. In one embodiment, the carrier molecule is a 16 aa peptide sequence derived from the homeodomain of *Antennapedia* (e.g. as sold under the name "Penetratin"), which can be coupled to a peptide via a terminal Cys residue. The "Penetratin" molecule and its properties are described in WO91/18981.

### A and P-type IPGs

Studies have shown that A-type mediators modulate the activity of a number of insulin-dependent enzymes such as cAMP dependent protein kinase (inhibits), adenylate cyclase (inhibits) and cAMP phospho-diesterases (stimulates). In contrast, P-type mediators modulate the activity of insulin-dependent enzymes such as pyruvate dehydrogenase phosphatase (stimulates), glycogen synthase phosphatase (stimulates), and cAMP dependent protein kinase (inhibits). The A-type mediators mimic the lipogenic activity of insulin on adipocytes, whereas the P-type mediators mimic the glycogenic activity of insulin on muscle. Both A-and P-type mediators inhibit cAMP dependent protein kinase and are mitogenic when added to fibroblasts in serum free media. The ability of the mediators to stimulate fibroblast proliferation is enhanced if the cells are transfected with the EGF-receptor. A-type mediators can stimulate cell proliferation in the chick cochleovestibular ganglia.

Soluble IPG fractions having A-type and P-type activity have been obtained from a variety of animal tissues including rat tissues (liver, kidney, muscle brain, adipose, heart) and bovine liver. A-type and P-type IPG biological activity has also been detected in human liver and placenta, malaria parasitized RBC and mycobacteria.

The ability of an anti-inositolglycan antibody to inhibit insulin action on human placental cytotrophoblasts and BC3H1 myocytes or bovine-derived IPG action on rat diaphragm and chick cochleovestibular ganglia suggests cross-species conservation of many structural features.

A-type substances are cyclitol-containing carbohydrates, also containing Zn²⁺ ions and optionally phosphate and having the properties of regulating lipogenic activity and inhibiting cAMP dependent protein kinase. They may also inhibit adenylate cyclase, be mitogenic when added to EGF-transfected fibroblasts in serum free medium. A-type IPGs isolated from sources such as human or bovine liver have the property of stimulating lipogenesis in adipocytes. In contrast, the A-type substances from porcine tissue have the properties of inhibiting lipogenesis and lowering blood glucose levels when administered to diabetics, i.e. patients or a suitable animal model.

P-type substances are cyclitol-containing carbohydrates, also containing Mn²⁺ and/or Zn²⁺ ions and optionally phosphate and having the properties of regulating glycogen metabolism and activating pyruvate dehydrogenase phosphatase. They may also stimulate the activity of glycogen synthase phosphatase, be mitogenic when added to fibroblasts in serum free medium, and inhibit cAMP dependent protein kinase.

Methods for obtaining A-type and P-type IPGs are set out in Caro et al, 1997, and in WO98/11116 and WO98/11117.

### Pharmaceutical Compositions

As mentioned above, GPI-PLD proteins including the variant proteins can used for treating diabetes and the complications of diabetes (e.g. insulin resistance), optionally in conjunction with other treatments for these disorders.

GPI-PLD proteins can be administered alone or in combination with other treatments for diabetes or diabetic complications, either simultaneously or sequentially. Examples of known diabetes treatments include (a) insulin, which is typically delivered by injection, (b) oral insulin compositions, (c) glucose sparing or insulin enhancing drugs, (d) a-glucosidase inhibitors to reduce carbohydrate absorption (precose and miglitol), and (e) drugs used to treat patients with insulin sensitivity, e.g. thiazolidinediones, such as Rezulin, rosiglitazone, piogliazone and tyrosine phosphatase inhibitors.

In further embodiments, the GPI-PLD can be administered with P and/or A-type IPGs, and/or antagonists of these substances. Methods for obtaining A-type and P-type IPGs and their antagonists are set out in Caro et al, 1997, and in WO98/11116 and WO98/11117.

The role of P and A-type IPGs and their use in the diagnosis and treatment of diabetes is disclosed in WO98/11435. In summary, this application discloses that in some forms of diabetes the ratio of P:A type IPGs is imbalanced and can be corrected by administering a medicament comprising the appropriate ratio of P or A-type IPGs or antagonist thereof. In particular, WO98/11435 describes the treatment of obese type II diabetes (NIDDM) patients with a P-type IPG or with an A-type IPG antagonist, such as antibodies which bind specifically to the A-type IPG, and the treatment of IDDM or lean type II diabetes (NIDDM) (body mass index < 27) with a mixture of A and P-type IPGs, typically in a P:A ratio of about 6:1 for males and 4:1 for females.

The compositions of the invention can be used in the treatment of diabetes, in particular insulin dependent forms of diabetes (type I and type II diabetes). They can also be used in the treatment of the complications of diabetes and in particular forms of insulin resistance such as insulin resistance in type I or type II diabetes and brittle diabetes.

In a further aspect, GPI-PLD proteins can used for treating liver dysfunction, optionally in conjunction with other treatments for these disorders. Preferably, the GPI-PLD is administered with apolipoprotein A1, and more preferably, as a complex with this substance. The isolation of apolipoprotein A1 is described in Hoener et al (1993), Deeg et al (1994) and Brewer et al (1986).
The compositions can be used to treat liver dysfunction conditions which are characterised by reduced levels of apolipoprotein A1 and/or GPI-PLD and/or apolipoprotein A1/GPI-PLD complex.

GPI-PLD proteins can be administered alone or in combination with other treatments for liver dysfunction, either simultaneously or sequentially.

GPI-PLD proteins and IPGs can be used for treating treatment of conditions caused by a product of an infectious organism which is capable of inhibiting GPI-PLD.

As mentioned above, in further embodiments, the GPI-PLD can be administered alone or in combination with P and/or A-type IPGs.

In all of the above embodiments, the GPI-PLD proteins and any accompanying compositions can be formulated in pharmaceutical compositions, which may comprise, in addition to one of the above substances, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, intraperitoneal routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection, lactated Ringer's injection. Preservatives, stabilisers, buffers, antioxidants and/or other additives may be included as required.

Whether it is a polypeptide, peptide, nucleic acid molecule, small molecule or other pharmaceutically useful compound of the invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980.

### GPI-PLD nucleic acid

"GPI-PLD nucleic acid" includes a nucleic acid molecule which has a nucleotide sequence encoding a polypeptide which includes the amino acid sequence shown in Figures 4 to 6, and in some embodiments of the invention extends to the known human liver and pancreatic forms of GPI-PLD (L11701 and L11702). These forms of GPI-PLD have been mapped to human chromosome 6 and are contained in the 4 centimorgan region of D6S1660-D6S1558 at positions 95.95 and 99.71 (NCBI GeneMap'98). The gene starts in the cytogenic region corresponding to 6p22.3 and extends into 6p21.3. This region also contains the IDDM1 and HLA loci (although the HLA genes map to the adjacent D6S1558-D6S1616 interval). The mouse GPI-PLD gene has also been mapped to chromosome 13, near the *fim 1* locus, which is found in humans on chromosome 6.

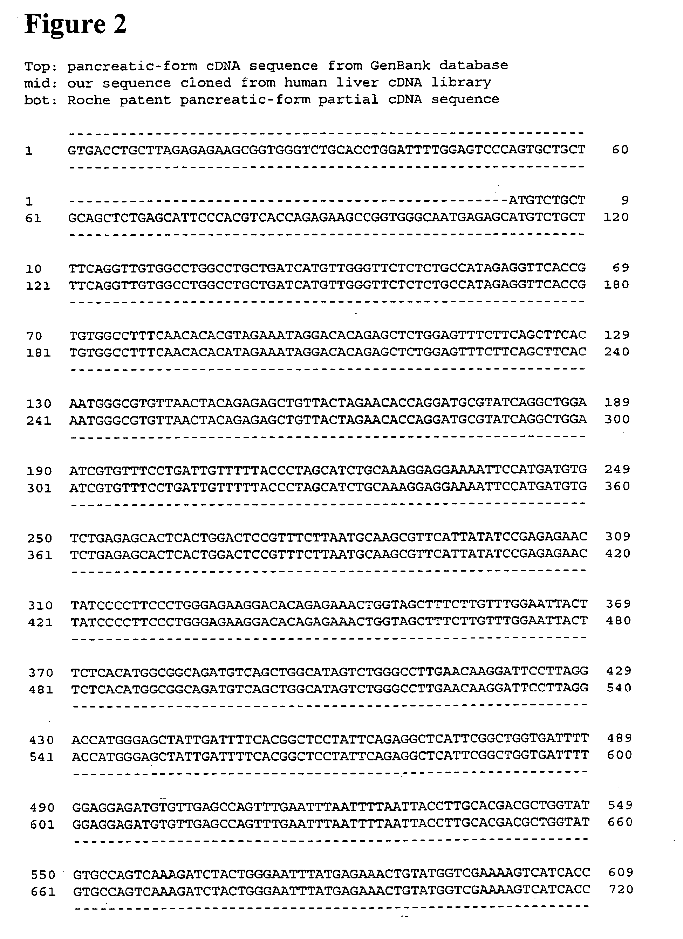

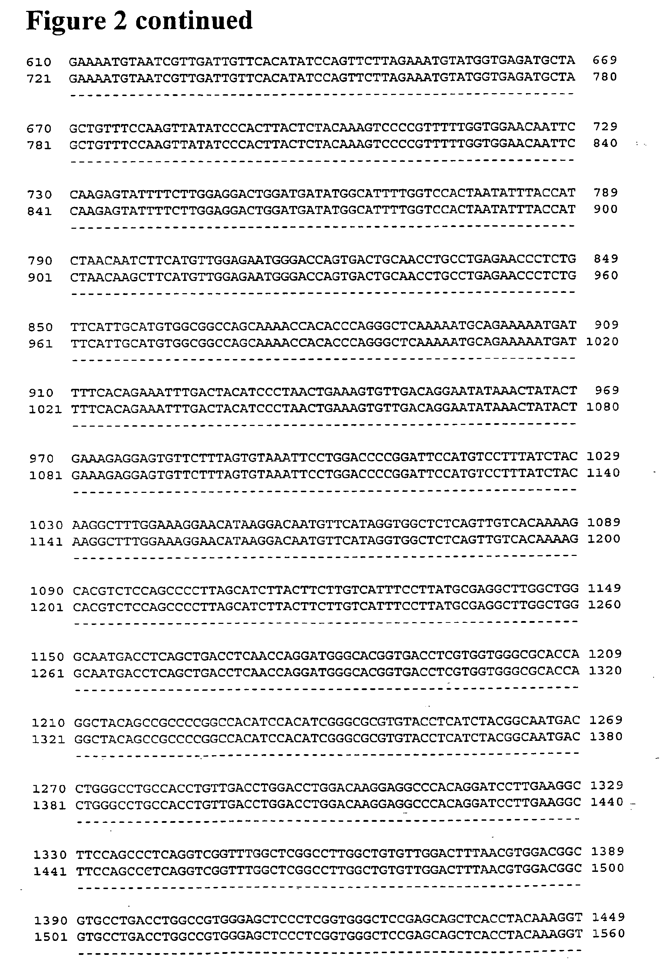

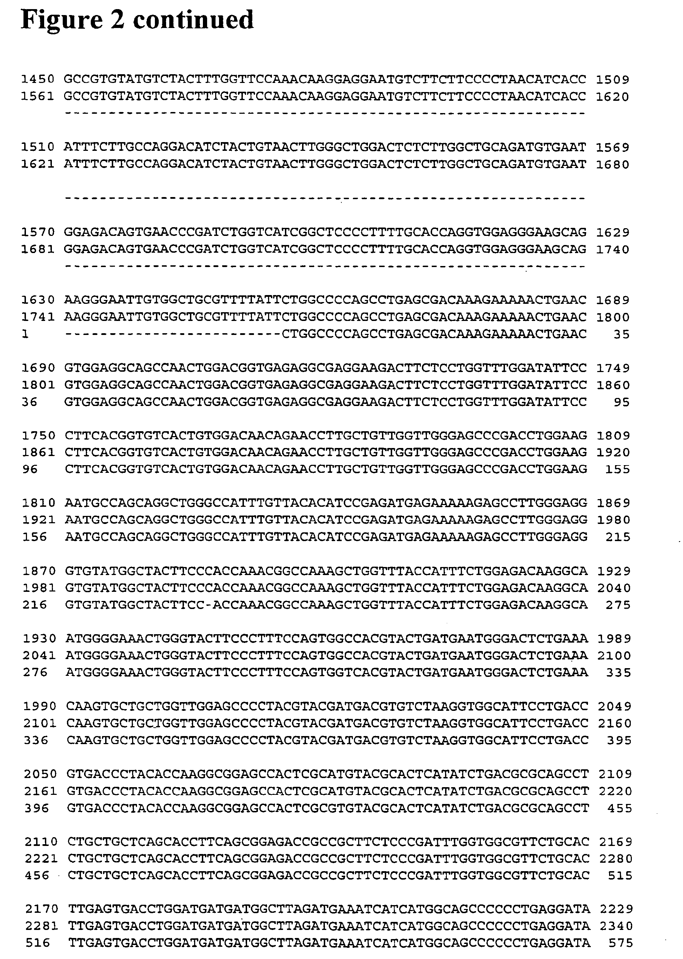

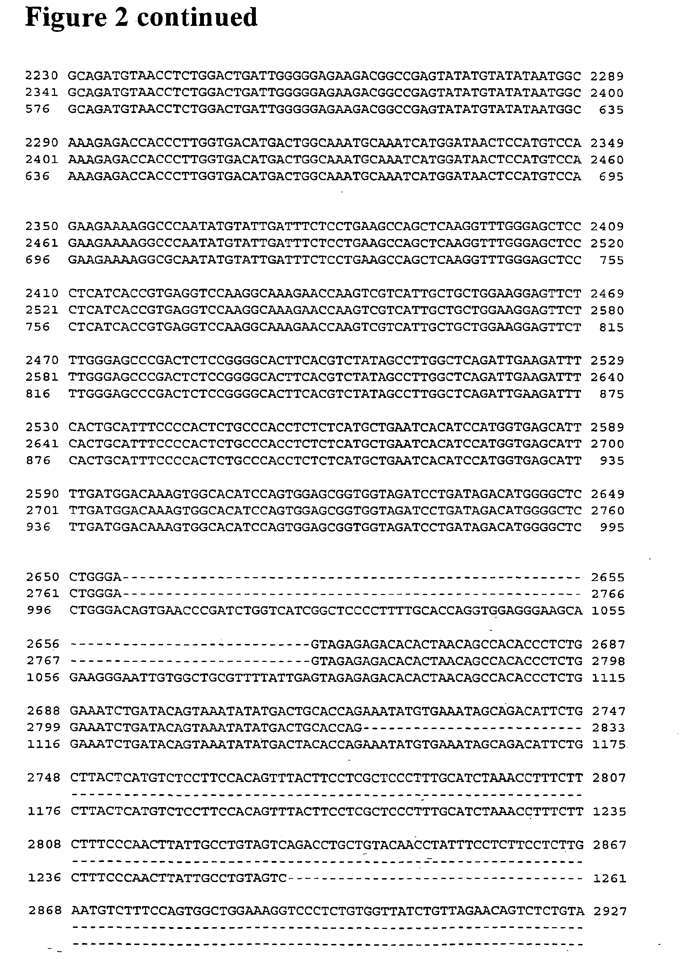

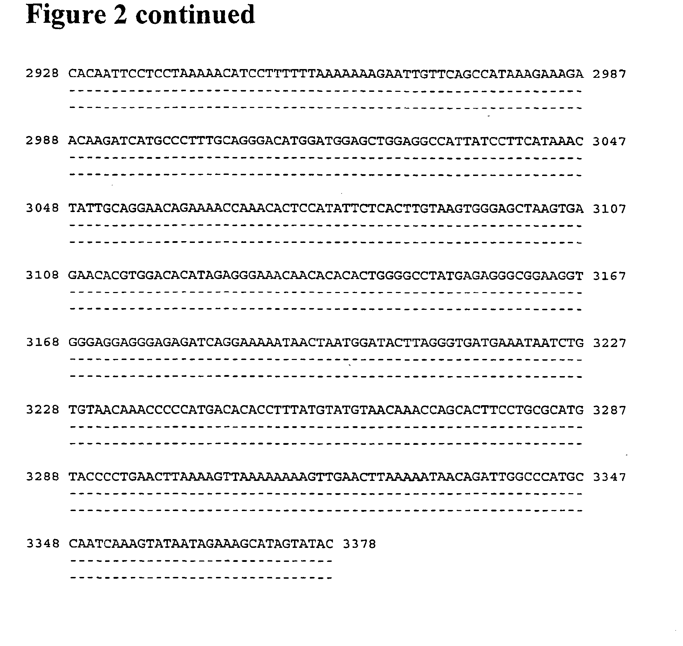

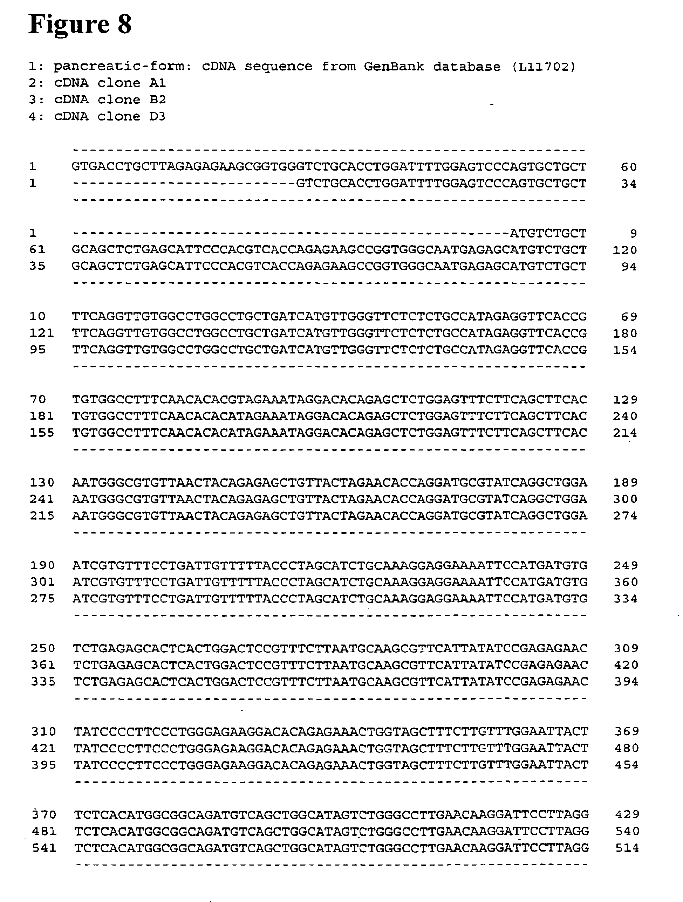

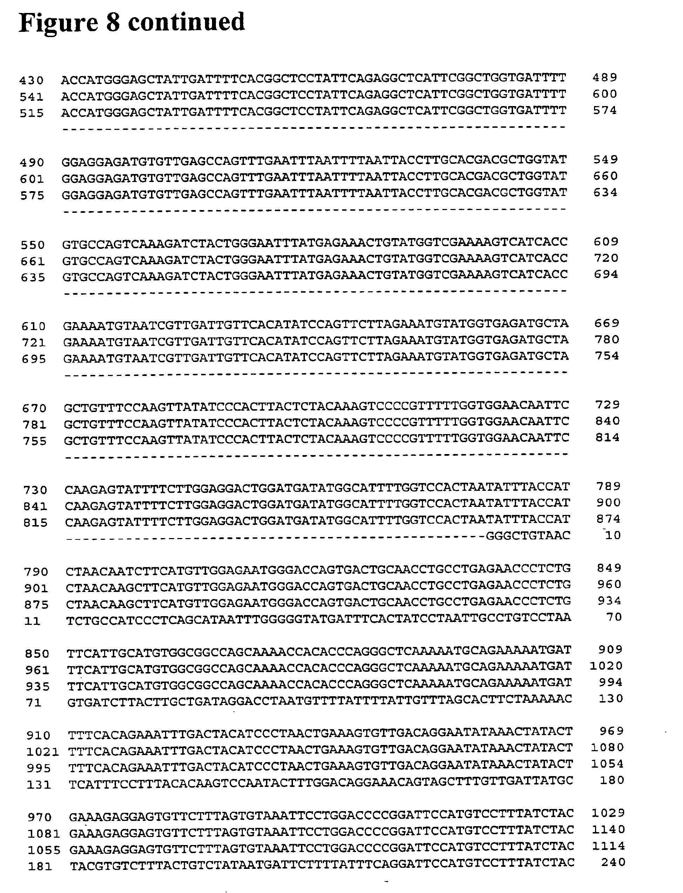

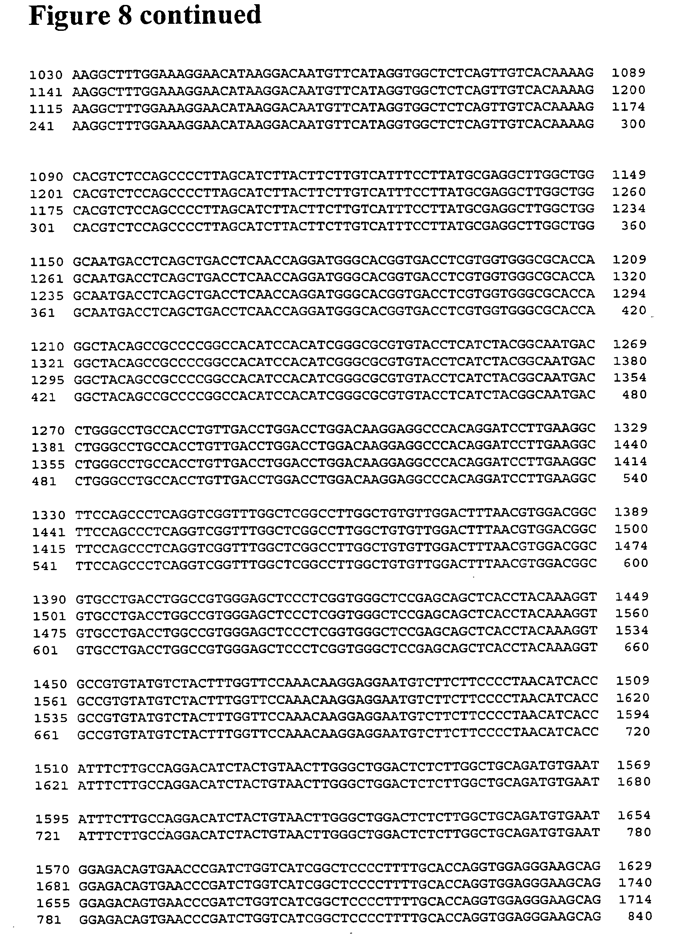

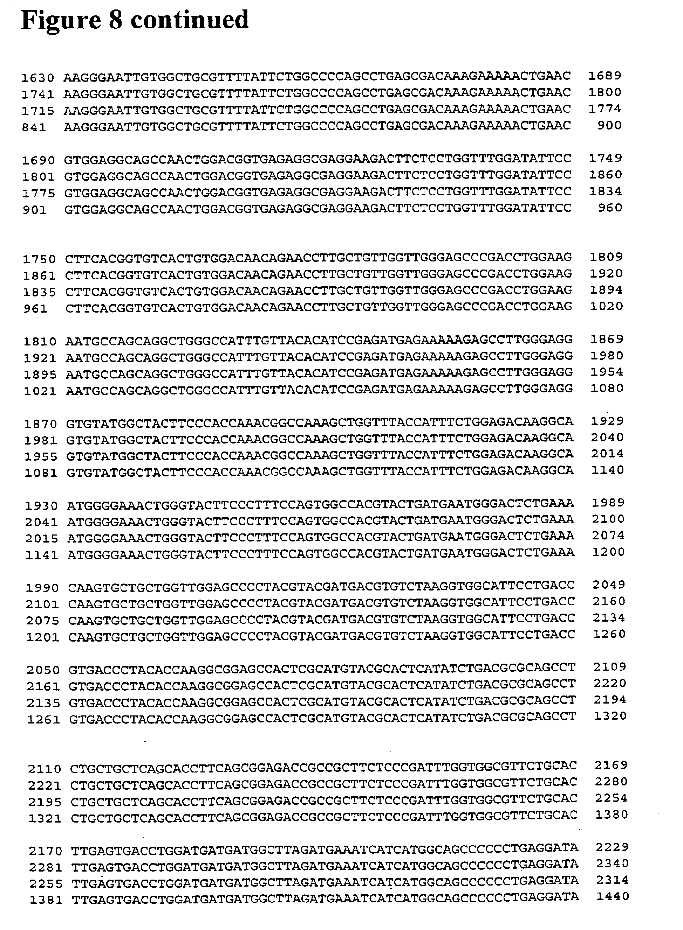

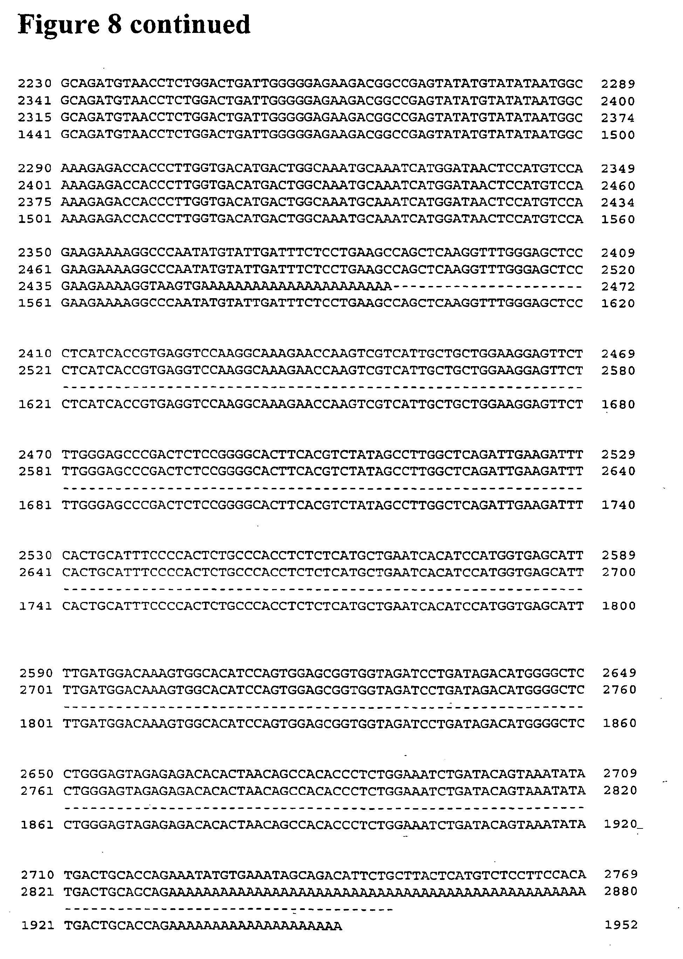

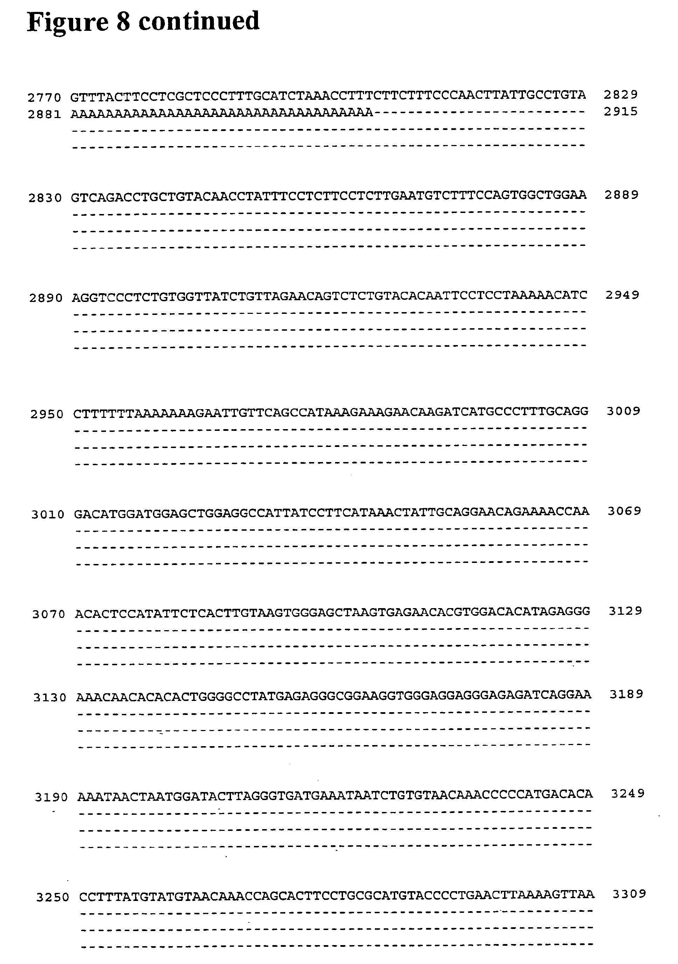

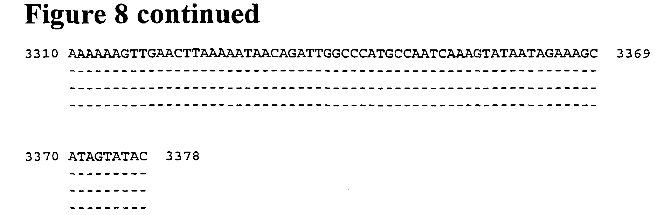

The GPI-PLD coding sequence may be that shown in Figures 2, 4 to 6 or 8, a complementary nucleic acid sequence, or it may be a mutant, variant, derivative or allele of these sequences. The sequence may differ from that shown by a change which is one or more of addition, insertion, deletion and substitution of one or more nucleotides of the sequence shown. Changes to a nucleotide sequence may result in an amino acid change at the protein level, or not, as determined by the genetic code.

The encoded polypeptide may comprise an amino acid sequence which differs by one or more amino acid residues from the amino acid sequence shown in the figures. Nucleic acid encoding a polypeptide which is an amino acid sequence mutant, variant or allele of the sequence shown in Figures 1, 3 or 7 is further provided by the present invention. Such polypeptides are discussed below. Nucleic acid encoding such a polypeptide may show greater than about 70% identity, greater than about 80% identity, greater than about 90% identity, greater than about 95% identity, greater than about 98% identity, or greater than about 99% identity with a sequence shown in the figures.

The present invention also includes fragments of the GPI-PLD nucleic acid sequences described herein, the fragments preferably being at least 12, 15, 30, 45, 60, 120 or 240 nucleotides in length.

Generally, nucleic acid according to the present invention is provided as an isolate, in isolated and/or purified form, or free or substantially free of material with which it is naturally associated, such as free or substantially free of nucleic acid flanking the gene in the human genome, except possibly one or more regulatory sequence(s) for expression. Nucleic acid may be wholly or partially synthetic and may include genomic DNA, cDNA or RNA. Where nucleic acid according to the invention includes RNA, reference to the sequence shown should be construed as reference to the RNA equivalent, with U substituted for T.

Nucleic acid sequences encoding all or part of the GPI-PLD gene and/or its regulatory elements can be readily prepared by the skilled person using the information and references contained herein and techniques known in the art (for example, see Sambrook, Fritsch and Maniatis, "Molecular Cloning, A Laboratory Manual, Cold Spring Harbor Laboratory Press, 1989, and Ausubel et al, Short Protocols in Molecular Biology, John Wiley and Sons, 1992). These techniques include (i) the use of the polymerase chain reaction (PCR) to amplify samples of such nucleic acid, e.g. from genomic sources, (ii) chemical synthesis, or (iii) amplification in *E. coli*. Modifications to the GPI-PLD sequences can be made, e.g. using site directed mutagenesis, to provide expression of modified GPI-PLD protein or to take account of codon preference in the host cells used to express the nucleic acid.

In order to obtain expression of the GPI-PLD nucleic acid sequences, the sequences can be incorporated in a vector having control sequences operably linked to the GPI-PLD nucleic acid to control its expression. The use of expression systems has reached an advanced degree of sophistication. The vectors may include other sequences such as promoters or enhancers to drive the expression of the inserted nucleic acid, nucleic acid sequences so that the GPI-PLD protein is produced as a fusion and/or nucleic acid encoding secretion signals so that the polypeptide produced in the host cell is secreted from the cell. GPI-PLD protein can then be obtained by transforming the vectors into host cells in which the vector is functional, culturing the host cells so that the GPI-PLD protein is produced and recovering the GPI-PLD protein from the host cells or the surrounding medium. Prokaryotic and eukaryotic cells are used for this purpose in the art, including strains of *E. coli*, yeast, and eukaryotic cells such as COS or CHO cells. The choice of host cell can be used to control the properties of the GPI-PLD protein expressed in those cells, e.g. controlling where the polypeptide is deposited in the host cells or affecting properties such as its glycosylation and phosphorylation.

PCR techniques for the amplification of nucleic acid are described in US Patent No:4,683,195. In general, such techniques require that sequence information from the ends of the target sequence is known to allow suitable forward and reverse oligonucleotide primers to be designed to be identical or similar to the polynucleotide sequence that is the target for the amplification. PCR comprises steps of denaturation of template nucleic acid (if double-stranded), annealing of primer to target, and polymerisation. The nucleic acid probed or used as template in the amplification reaction may be genomic DNA, cDNA or RNA. PCR can be used to amplify specific sequences from genomic DNA, specific RNA sequences and cDNA transcribed from mRNA, bacteriophage or plasmid sequences. The GPI-PLD protein nucleic acid sequences provided herein readily allow the skilled person to design PCR primers. References for the general use of PCR techniques include Mullis et al, Cold Spring Harbor Symp. Quant. Biol., 51:263, 1987; Ehrlich (ed), PCR Technology, Stockton Press, NY, 1989; Ehrlich et al, Science, 252:1643-1650, 1991; "PCR protocols; A Guide to Methods and Applications", Eds. Innis et al, Academic Press, New York, 1990.

Nucleic acid according to the present invention is obtainable using one or more oligonucleotide probes or primers designed to hybridize with one or more fragments of the nucleic acid sequence shown in the figures, particularly fragments of relatively rare sequence, based on codon usage or statistical analysis. A primer designed to hybridize with a fragment of the nucleic acid sequence shown in the above figures may be used in conjunction with one or more oligonucleotides designed to hybridize to a sequence in a cloning vector within which target nucleic acid has been cloned, or in so-called "RACE" (rapid amplification of cDNA ends) in which cDNA's in a library are ligated to an oligonucleotide linker and PCR is performed using a primer which hybridizes with a GPI-PLD nucleic acid sequence shown in figures and a primer which hybridizes to the oligonucleotide linker.

Such oligonucleotide probes or primers, as well as the full-length sequence (and mutants, alleles, variants and derivatives) are also useful in screening a test sample containing nucleic acid for the presence of alleles, mutants and variants, especially those that lead to the production of inactive forms of GPI-PLD protein, the probes hybridizing with a target sequence from a sample obtained from the individual being tested. The conditions of the hybridization can be controlled to minimise non-specific binding, and preferably stringent to moderately stringent hybridization conditions are preferred. The skilled person is readily able to design such probes, label them and devise suitable conditions for the hybridization reactions, assisted by textbooks such as Sambrook et al (1989) and Ausubel et al (1992).

Examples of "stringent conditions" are those which: (1) employ low ionic strength and high temperature for washing, for example 0.015 M sodium chloride/0.0015 M sodium citrate/0.1% sodium dodecyl sulphate at 50°C; (2) employ during hybridisation a denaturing agent, such as formamide, for example, 50% (v/v) formamide with 0.1% BSA/0.1% Ficoll/0.1% polyvinylpyrrolidone/50mM sodium phosphate buffer at pH 6.5 with 750mM sodium chloride, 75mM sodium citrate at 42°C; or (3) employ 50% formamide, 5 x SSC (0.75 M NaCl, 0.075 M sodium citrate), 50mM sodium phosphate (pH 6.8), 0.1% sodium pyrophosphate, 5 x Denhardt's solution, sonicated salmon sperm DNA (50mg/ml), 0.1% SDS, and 10% dextran sulphate at 42°C, with washes at 42°C in 0.2 x SSC and 50% formamide at 55°C, followed by high stringency wash consisting of 0.1 x SSC containing EDTA at 55°C. These hybridisation conditions may be used in the context of defining nucleic acid sequences which hybridize with GPI-PLD nucleic acid sequences.

### Uses of GPI-PLD Nucleic Acid

The GPI-PLD nucleic acid sequences can be used in the preparation of cell lines capable of expressing GPI-PLD and included in expression vectors or otherwise formulated, e.g. for use in gene therapy techniques.

Thus, the present invention provides a cell line for transplantation into a patient, the cell line being transformed with nucleic acid encoding GPI-PLD, and being capable of expressing and secreting GPI-PLD. In one embodiment, the cell lines are encapsulated, e.g. in a biocompatible polymer, so that the GPI-PLD produced by the cell line can be secreted into the patient, while preventing rejection by the immune system of the host. Methods for encapsulating cells in biocompatible polymers are described in WO93/16687 and WO96/31199.

As a further alternative, the nucleic acid encoded the GPI-PLD protein could be used in a method of gene therapy, to treat a patient who is unable to synthesize the active polypeptide or unable to synthesize it at the normal level, thereby providing the effect provided by wild-type GPI-PLD protein and suppressing the occurrence of diabetes in the target cells.

Vectors such as viral vectors have been used in the prior art to introduce genes into a wide variety of different target cells. Typically, the vectors are exposed to the target cells so that transfection can take place in a sufficient proportion of the cells to provide a useful therapeutic or prophylactic effect from the expression of the desired polypeptide. The transfected nucleic acid may be permanently incorporated into the genome of each of the targeted tumour cells, providing long lasting effect, or alternatively the treatment may have to be repeated periodically.

A variety of vectors, both viral vectors and plasmid vectors, are known in the art, see US Patent No: 5,252,479 and WO93/07282. In particular, a number of viruses have been used as gene transfer vectors, including papovaviruses, such as SV40, vaccinia virus, herpesviruses, including HSV and EBV, and retroviruses. Many gene therapy protocols in the prior art have used disabled murine retroviruses.

As an alternative to the use of viral vectors other known methods of introducing nucleic acid into cells includes electroporation, calcium phosphate co-precipitation, mechanical techniques such as microinjection, transfer mediated by liposomes and direct DNA uptake and receptor-mediated DNA transfer.

As mentioned above, the aim of gene therapy using nucleic acid encoding the GPI-PLD protein, or an active portion thereof, is to increase the amount of the expression product of the nucleic acid in cells in which the level of the wild-type GPI-PLD protein is absent or present only at reduced levels. Target cells for gene therapy include insulin secreting b-cells or any neuron derived cells. Cell engineering can be used to provide the overexpression or repression of GPI-PLD protein in transfected cell lines which can then be subsequently transplanted to humans. Gene therapy can be employed using a promoter to drive GPI-PLD protein expression in a tissue specific manner (i.e. an insulin promoter linked to GPI-PLD cDNA will overexpress GPI-PLD protein in b-cells and transiently in the brain). If defective function of GPI-PLD protein is involved in neurological disease, GPI-PLD protein can be overexpressed in transformed cell lines for transplantation.

Gene transfer techniques which selectively target the GPI-PLD nucleic acid to target tissues are preferred. Examples of this included receptor-mediated gene transfer, in which the nucleic acid is linked to a protein ligand via polylysine, with the ligand being specific for a receptor present on the surface of the target cells.

### Diagnostic Methods

Methods for determining the concentration of analytes in biological samples from individuals are well known in the art and can be employed in the context of the present invention to determine the presence or amount of GPI-PLD or a product of GPI-PLD action in a biological sample from a patient. This in turn can allow a physician to determine whether a patient suffers from one of the conditions discussed above and so optimise the treatment of it.

As discussed above, the conditions include diabetes and diabetic complications, liver dysfunction or disorders involving pancreatectomies, and conditions mediated by a product of an infectious organism which is capable of inhibiting GPI-PLD, such as septic shock.

Broadly, the methods divide into those screening for the presence of GPI-PLD protein nucleic acid sequences and those that rely on detecting the presence or absence of the GPI-PLD protein polypeptide or a product of GPI-PLD action (e.g. IPGs or acyl-IPGs). The methods make use of biological samples from individuals that are suspected of contain the nucleic acid sequences or polypeptide.

These diagnostic methods can employ biological samples such as blood, serum, tissue samples or urine. In view of the fact that the activity of GPI-PLD is thought to be due to the level of the enzyme circulating in serum, the use of serum or blood samples is preferred.

The assay methods for determining the amount or concentration of GPI-PLD protein typically either employ binding agents having binding sites capable of specifically binding to GPI-PLD or the product of GPI-PLD action in preference to other molecules or measure a characteristic biological activity of GPI-PLD. Examples of binding agents include antibodies, receptors and other molecules capable of specifically binding the enzyme. Conveniently, the binding agent(s) are immobilised on solid support, e.g. at defined locations, to make them easy to manipulate during the assay.

In one format

GPI-PLD can be determined in a method which disclosed herein comprises the steps of:
(a) contacting a biological sample obtained from the patient with a solid support having immobilised thereon a binding agent having binding sites specific for GPI-PLD or a product of GPI-PLD action;
(b) contacting the solid support with one or more labelled developing agents capable of binding to unoccupied binding sites, bound GPI-PLD or product, or occupied binding sites; and,
(c) detecting the label of the developing agents specifically binding in step (b) to obtain a value representative of the amount of GPI-PLD or the product of GPI-PLD action in the sample.

Alternatively or additionally, the method can assess GPI-PLD levels by measuring one of its biological activities, which are discussed further below.

The products of GPI-PLD action include acyl-IPGs and IPGs, the characteristic activities of which are discussed above. Antibodies which are capable of binding to IPGs are disclosed in WO98/1116, WO98/11117 and WO99/47565.

The sample is generally contacted with the binding agent(s) under appropriate conditions so that GPI-PLD present in the sample can bind to the binding agent(s). The fractional occupancy of the binding sites of the binding agent(s) can then be determined using a developing agent or agents. Typically, the developing agents are labelled (e.g. with radioactive, fluorescent or enzyme labels) so that they can be detected using techniques well known in the art. Thus, radioactive labels can be detected using a scintillation counter or other radiation counting device, fluorescent labels using a laser and confocal microscope, and enzyme labels by the action of an enzyme label on a substrate, typically to produce a colour change. The developing agent(s) can be used in a competitive method in which the developing agent competes with the analyte for occupied binding sites of the binding agent, or non-competitive method, in which the labelled developing agent binds analyte bound by the binding agent or to occupied binding sites. Both methods provide an indication of the number of the binding sites occupied by the analyte, and hence the concentration of the analyte in the sample, e.g. by comparison with standards obtained using samples containing known concentrations of the analyte.

### Experimental

In one embodiment, the present invention is based on the realisation that GPI-PLD is responsible for the production of IPG second messengers following binding of insulin to its receptor. The IPGs then interact with other cellular enzymes instigating some of the metabolic effects of the hormone. In view of this, insulin resistance may be caused by deficiencies in GPI-PLD; it has shown that pancreatic islet cells produce and secrete GPI-PLD, which is transported in blood complexed with apolipoprotein A1, and may therefore represent the major source of circulating enzyme. If this is indeed the case then the insulin resistance seen in early type I diabetes mellitus (IDDM) may result from decreased circulating GPI-PLD levels. This may have direct therapeutic relevance in that co-infusion of insulin with GPI-PLD may in fact be a far more effective therapy for diabetic patients than insulin.

In a further embodiment, the present invention is based on the realisation that GPI-PLD can be used in the treatment of liver dysfunction, and in particular combination with apoliprotein A1 to which it is bound in human serum and blood. As GPI-PLD is transported in blood complexed with apolipoprotein A1, liver dysfunction, and especially dysfunction characterised by reduced apolipoprotein A1 levels, can be treated using GPI-PLD.

In a third embodiment, the present invention is based on the observation that in conditions such as septic shock, endotoxin is released from the microorganisms causing sepsis, leading to the clinical symptoms of septic shock such as total organ failure and fatal shock. The endotoxins can be glycolipids released from gram negative bacteria or glycolipids such as LAM released from *Mycobacteria* such as *Tuberculosis*. Without wishing to be bound by any particular theory, these endotoxins are believed to act by inhibiting GPI-PLD.

### Screening of human liver cDNA library

A human liver cDNA library (Gibco BRL, cat # 10422-012, lot # HF4703) was screened for GPI-PLD, resulting in the isolation of 3 cDNA clones. The nucleic acid sequences of the clones are shown in Figures 4 to 6, with the deduced amino acid sequences shown in Figure 3.

Clone a1 represents the full length cDNA. There are only two differences within the coding region of this sequence when compared to that of the human GPI-PLD pancreatic form described in the GenBank database (accession number L11702). These are a *g* to *a* conversion at positions 88 (L11702), 199 (a1) and a *t* to *g* conversion at positions 797 (L11702), 908(a1). Interestingly this latter conversion creates a unique *Hind*III restriction site in the al clone. Both conversions result in amino acid differences, the first changes amino acid 30 from a valine in L11702 to an isoleucine in a1, and the second changes amino acid 266 from an isoleucine in L11702 to a serine in a1. Clone a1 also differs from L11702 in that it contains 5' untranslated region (UTR) and only shares the first 168 bases of the 3' UTR before terminating in a poly-A tail.

Clone b2 lacks exons 23-25 of GPI-PLD, which begins at position 2469 in the a1 nucleotide sequence. However, the sequence from here to the end of b2 (2444-2473) does not contain a stop codon. It is therefore not clear whether b2 represents a cDNA with a different final exon or is the produce of aberrant processing.

Clone d3 shared the 3'coding and 3'UTR sequence of the a1 clone from position 1119 onwards, however the initial 1008 base pairs of coding sequence representing the initial 12 exons, are absent from this clone. Clone d3 contains a methionine initiation codon in frame to the coding sequence at position 202 and a unique 5' UTR. Translation of d3 from this codon would result in a unique sequence of 6 amino acids (1-6). Clone d3 therefore appears to represent a true transcript, in that it contains initiation and stop-codons and both 5' and 3' UTRs. The predicted protein product of this transcript would apparently lack the catalytic domain, which has been localised to the N-terminus of the GPI-PLD enzyme (amino acids 1-375), however the 4 EF hand-like domains would still be present.

Huang et al and Tsang et al (1992) reported that two variants or isoenzymes of GPI-PLD exist, the so-called liver and pancreatic forms (accession numbers L11701 and 11702). Other workers have detected L11702 cDNAs in human breast, eye, spleen, tonsil, and pancreas, as well as in liver. However, we failed to detect the liver form of GPI-PLD in the liver or in any other tissues.

### Gene mapping and localisation

The chromosomal gene isolated in the experiments above is over 100 kb in length. The gene was also isolated on a PAC and mapped by fluorescence-in situ hybridisation (FISH) to 6p22.3 extending into 6p21.3, agreeing with recent radiation hybrid maps as seen on GeneMap'98; NCBI). The IDDM1 susceptibility gene also maps to 6p21.3, although recent evidence suggests that at least two closely-linked loci for IDDM1 are in the MHC region. The MHC locus itself seems to map to a region adjoining the GPI-PLD locus rather than within the same microsatellite band, so the significance of the proximity of the GPI-PLD and IDDM1 loci is unclear.

Northern blots of the mRNA species found in liver have shown two presumed splice variants as well as the full-length transcript. One has a deletion of about 160 amino acids from the mature 817 amino acid protein. The other seems to be a C-terminal deletion, which may therefore be non-functional if other authors are correct in finding that the C-terminus is necessary for enzyme activity.

The predominant GPI-PLD species detected after tissue extraction by antibodies (Western blots) has apparent molecular weight of about 47 kD, which agrees with other authors that full-length GPI-PLD is taken up from the plasma and processed to smaller active species.

### PCR Analysis of GPI-PLD isoforms

PCR was used to compare the expression of putative cDNAs L11701 and L11702 using oligos pairs in cDNA made from human liver mRNA or in genomic DNA. cDNA synthesis reactions from which reverse transcriptase was omitted served as negative controls.

Two regions of the cDNAs were found to have a sufficient number of base differences to enable the synthesis of isoform-specific oligonucleotides. Region 1 contained 6 base pair changes over a total length of 25 nucleotides. From this region two isoform-specific reverse oligos were made:
P2 cagc**a**g**a**ggctgc**g**cgtc**a**gat**a**tg (L11702: 2115-2091)
L2 cagc**g**g**t**ggctgc**a**ggtc**g**gat**g**tg (L11701: 2150-2126)

These were matched with forward oligos for gc content from a region approximately 700bp upstream. This region is shown below with differences highlighted in bold and the oligo sequences underlined:
P1 **g**tgttggactttaac**g**tgga**c**ggcgtgcctgacctggccg
   (L11702: 1366-1405)
L1 **a**tgttggactttaac**a**tgga**t**ggcgtgcctgacctggccg
   (L11701: 1400-1440)

Region 2 (1 (L11701; L11702) contained 9 base pair changes over a total length of 32 nucleotides and was used to make two isoform-specific reverse oligos as before:
P19 **gtac**gt**a**ggggctcc**a**accagcagcac**t**tg**tt**(L11702: 2019-1988)
L4 **acgt**gt**c**ggggctcc**c**accagcagcac**c**tg**gg**(L11701: 2054-2023)

These oligos were paired with a single oligo which recognizes both isoforms approximately 300bp upstream which would also enable PCR from genomic DNA:
U2 tggttgggagcccgacctggaagaatgccagc
(L11702: 1787-1818; L11701: 1822-1853)

5mg total human liver RNA (Invitrogen) was reverse transcribed using Superscript II (GibcoBRL) for 90 mins in a total volume of 35ul. Negative controls contained 5mg of RNA but no reverse transcriptase (lanes 2, 4, 6 and 9). 2.5ml of this reaction or 888ng of human genomic DNA (Promega) was transferred to a 50ml PCR reaction containing 25pmoles of each oligo. After an initial 4-min 94°C denaturing cycle, 30 cycles were performed (25 secs denaturing - 94°C, 30 secs annealing, 30 secs extension - 72°C) and PCR products resolved on a 1% agarose gel. Annealing temperatures of the oligo pairs were as follows: P1 & P2 - 62°C; L1 & L2 - 66°C; U2 & P19 - 68.3°C; U2 & L4 - 71.5°C).

### Southern Blot

A Southern blot of PAC 282J10 DNA and human genomic DNA was hybridised with a cDNA probe containing exons 15-19. The same bands hybridise in both PAC and genomic DNA therefore suggesting that only one copy of the GPI-PLD gene is present in the human genome. This result is in accord with the finding of only one gene in the mouse. (LeBoeuf et al, Mammalian Genome 9:710-714, 1998).

4mg of human genomic DNA or 1mg of PAC 282J10 DNA was digested with the restriction enzymes ApaI, EcoRI or NsiI (Promega) at 37°C overnight and run on 1% agarose gel, which was denatured, neutralised and blotted in 20XSSC overnight. DNA was UV crosslinked onto the blot and then hybridised with ³²P-labelled P1/P2 PCR product. The blot was then washed with decreasing SSC concentrations, the final wash being 0.2XSSC, 0.1%SDS for 20 mins at 65°C. Autoradiographs were exposed at -80°C for 1h (282J10) or 3 days (genomic).

### GPI-PLD gene structure

The structure of the human GPI-PLD gene has been determined. It comprises 25 exons and extends over more than 100 kb of chromosome 6p22.3 into 6p21.3. We have used Southern blot analysis to determine that only one GPI-PLD gene exists in the human genome.

Using PCR analysis as described above, we have been unable to prove the existence of the so-called liver form of GPI-PLD (GenBank accession number L11701), whereas the so called pancreas form (L11702) is the form we have detected in human liver. These data show that the two forms do not exist alongside each other in the human liver, however it is still possible that L11701 represents a polymorphic variant not seen in the subjects from whom our liver RNA was obtained.

### GPI-PLD gene expression

Using PCR we have compared the expression of GPI-PLD in cDNA libraries made from human tissues. GPI-PLD appears most abundant in the liver followed by the lung. A very low level of expression was seen in kidney and heart and skeletal muscle, however we were unable to detect expression in pancreas, brain or placenta.

Recombinant GPI-PLD has been purified from stable CHO cell lines transfected with the full-length human GPI-PLD cDNA clone a1 isolated previously from a human liver cDNA library. Recombinant GPI-PLD cleaves the GPI substrate mfVSG, and like its counterpart purified from serum, this action is inhibited by prior incubation with the transition metal ion chelator 1,10-phenanthroline.

We have identified at least two systems which do not appear to express the GPI-PLD gene, namely the human placenta and the rat basophil-like cell line RBL2H3. However in both cases abundant GPI-PLD protein and enzyme activity is detectable, thus confirming our prediction that in tissues which do not express the gene, protein is still expressed and is presumably uptaken from the vast reserves found in serum. Experiments using the mouse skeletal muscle cell line C2C12 indicate that over 70% of the GPI-PLD activity present within the cells is derived from serum.

### GPI-PLD obtained from serum by cells is required for second messenger signalling

The principle goal of these experiments was to determine the role of glycosylphosphatidylinositol phospholipase D (GPI-PLD) in a type one hypersensitivity reaction. This reaction involved the cross-linking of IgE receptors on the mast cell surface, leading to the release of allergic mediators.

Such an allergic reaction has been experimentally reproduced in our laboratory, using a rat basophilic leukaemia cell line, RBL-2H3. These cells naturally have unoccupied IgE receptors (FceR1, or high-affinity receptors), allowing them to be passively sensitised with an IgE isotype of choice.

RBL-2H3 cell culture was maintained in Eagles minimum essential medium, containing 10% Foetal Bovine Serum (FBS) (heat activated), 100 U/ml Penicillin, 100 mg/ml Streptomycin and 2 mM L-glutamine.

Previous research indicates that RBL-2H3 cells derive their GPI-PLD from the culture serum (data not shown). Therefore, it follows that inactivation of this external source of GPI-PLD would deprive the cells of any further enzyme.

Inactivation of GPI-PLD activity in foetal bovine serum was achieved according to the method of Kung et al (Biochimica et Biophysica Acta, 1357:329-338, 1997). Briefly, FCS was adjusted to pH 11 using concentrated hydrochloric acid, and incubated for 1 hour at 37°C using. After this time, the pH was adjusted to 7.4, and GPI-PLD activity was determined using an enzymatic assay (Davitz et al, J. Biol. Chem., 264:13760-13764, 1989). Results indicated that this alkaline incubation severely depleted GPI-PLD activity (data not shown).

To determine the effect of culture of RBL-2H3 cells in GPI-PLD inactive serum, the supplemented MEM was replaced with MEM in which the FBS had been inactivated. Although the cell appearance was not dramatically altered by the altered culture conditions, determination of GPI-PLD activity showed a dramatic reduction in activity.

### GPI-PLD activity in cells cultured with GPI-PLD active/inactive FBS:

Active = 0.66 units GPI-PLD activity/mg of protein.

Inactive = 0.11 units GPI-PLD activity/mg of protein.

The effect of a reduced GPI-PLD activity on the cell's ability to respond to IgE cross-linking was determined as follows:
RBL-2H3 cells were grown to confluence, after which time the adherent cells were removed from the culture flask using a cell scraper. The cell density was determined, using a haemocytometer, and adjusted to 2 x 10⁵ per ml. The cells were seeded at 1 ml per well in a 24 well culture plate and cultured for overnight at 37°C in a humidified 5% CO₂ incubator.

The overnight culture media was aspirated and replaced with fresh media containing Rat IgE anti-DNP 3mg/ml. After a 2 hour incubation period, the media was aspirated, and the cells were washed twice, with HEPES Buffered Saline. Cross-linking was achieved by the addition of 200 ml of DNP-Albumin at 100 ng/ml, and incubation for 2 hours. Mediator release was determined using a colorimetric assay to detect the presence of b-hexosaminidase and compared with the total cell b-hexosaminidase content (as determined by incubation with 200 ml 5% Triton X-100 detergent). (Yasuda et al, Int. Imunol., 7:251-258, 1995). As shown in the table below, the responsiveness to cross-linking was significantly reduced in those cells that were cultured in GPI-PLD inactive media.

### Percentage release in IgE linking activity assay (compared with total):

Active GPI-PLD culture = 48.79%.

Inactive GPI-PLD culture = 5.07%

### Phosphorylation of GPI-PLD

The phosphorylation state of the GPI-PLD enzymes was determined using MALDI-TOF mass spectrometry as described by Yip & Hutchins (1992). Spectrums of tryptic digests of the proteins can be compared before and after treatment with calf intestinal alkaline phosphatase. The specific kinases responsible for phosphorylation of GPI-PLD can then be determined by incubation of the GPI-PLD tryptic fragments with ATP in the presence of various kinases. Motif analysis of the amino acid sequence of human GPI-PLD using the HGMP "motif" package has revealed the presence of numerous potential phosphorylation sites for the enzymes cAMP-dependent protein kinase A, protein kinase C and protein kinase ck2 (formerly known as casine kinase II). Of these sites we have found that the site at amino acids 689-692 is a key site which when phosphorylated, e.g. by PKA, inhibits GPI-PLD biological activity.

These enzymes may therefore be involved in the activation/inactivation of GPI-PLD. Intriguingly the activity of protein kinase ck2 has been shown to be modulated by IPGs (Alemany et al, 1990) and there is also indirect evidence suggesting that IPGs may act through protein kinase C, thus suggesting the possibility of feedback loops regulating the production of IPGs.

### Discussion

GPI-PLD is a metalloenzyme with 5 and 10 atoms per molecule of calcium and zinc, respectively. It circulates in a complex with apolipoprotein A1. GPI-PLD is produced in the pancreas by both a and b-cells in the islets of Langerhans. It is also produced by a mouse insulinoma cell line (bTC3), with GPI-PLD and insulin generally colocalised intracellularly. The enzyme was shown to be secreted in response to insulin secretagogues. Both isoenzymes of GPI-PLD also seem to be present in liver; a major part of the activity could be washed away from the tissue by extraction with detergent-free buffer (thus, likely to be the plasma enzyme). There is some suggestions that the liver, as well as the pancreas, may contribute to the serum pool of GPI-PLD as patients with liver disease have lower levels of active enzyme, which is correlated with the reduced albumin levels.

It has been shown that streptozotcin-induced diabetes mellitus in the rat reduced the basal content of insulin-sensitive IPG in isolated hepatocytes by about 60%. The authors conclude that insulin resistance in these rats is related to the impairment of IPG metabolism. It has also been shown that the mRNA for a GPI-PLD-like gene was over expressed in genetically obese (ob/ob) mice in comparison to lean litter mates. In the context of the invention described herein, this latter finding suggests that GPI-PLD levels are responsive to the obese/diabetic genotype.

### References:

Huang et al, US Patent No: 5,418,147.
Tsang et al, FASEB J. (supp), 6:A1922, 1992.
Scallon et al, Science, 252:446-448, 1991.
Hoener et al, Eur. J. Biochem., 206:747-757, 1992.
Li et al, J. Biol. Chem., 269:28963-28971, 1994.
Heller et al, Eur. J. Biochem., 224:823-833, 1994.
Jones et al, Biochem. Biophys. Res. Comm., 233:432-437, 1997.
Rademacher et al, Brazilian J. Med. Biol. Res., 27:327-341, 1994.
Lin et al, J. Cell Biol., 115:220a, 1991
Thompson et al, Nucleic Acid Research, 22:4673-4680, 1994, with algorithm from Higgins et al, CABIOS, 8(2):189-191, 1992.
Alemany et al, Nature, 330:77-79, 1987.
Caro et al, Biochem. Molec. Med., 61:214-228, 1997.
Deeg & Verchere, Endocrinology, 136:819-826, 1997.
LeBoeuf et al, Mammalian Genome 9:710-714, 1998.

## Claims

1. An isolated glycosylphosphatidyl inositol specific phospholipase D (GPI-PLD) for use in therapy, wherein the GPI-PLD polypeptide comprises:
(a) a GPI-PLD polypeptide having an amino acid sequence as set out in Figure 1 or 3; or
(b) a GPI-PLD polypeptide having greater than 70% amino acid sequence identity with the amino acid sequence as defined in (a) that retains the enzymatic activity of cleaving the phosphodiester bond linking GPI to phosphatidic acid; or
(c) a GPI-PLD polypeptide which is an active portion of the GPI-PLD polypeptide as set out in (a); or
(d) a GPI-PLD polypeptide as defined in (a), (b) or (c) which is linked to a coupling partner.

2. The GPI-PLD according to claim 1, wherein the GPI-PLD is simultaneously or sequentially administered with apoliprotein A1, a P- and/or A-type inositolphosphoglycan (IPG) and/or an IPG antagonist.

3. The GPI-PLD polypeptide according to claim 1, having the insertion, addition, deletion or substitution of between 1 and 10 amino acids as compared to an amino acid sequence as set out in Figure 1 or 3.

4. The GPI-PLD according to any one of claims 1 to 3, wherein the coupling partner is an effector, a label, a drug, a toxin, a carrier or a transport molecule.

5. The GPI-PLD polypeptide according to any one of the preceding claims, wherein the GPI-PLD differs in amino acid sequence at one or more residues in the region corresponding to amino acid 689-692 inclusive (RRFS) of human GPI-PLD having the amino acid sequence shown in Figure 1.

6. The GPI-PLD polypeptide according to claim 5, which comprises a substitution in the region corresponding to amino acids 689-692 of human GPI-PLD having the amino acid sequence shown in Figure 1.

7. The GPI-PLD according to claim 6, wherein the substitution changes the serine residue at position 692 to an amino acid other than serine or threonine.

8. An isolated nucleic acid molecule encoding a GPI-PLD polypeptide according to any one of claims 1 to 7 for use in therapy.

9. The nucleic acid molecule according to claim 8, having the nucleic acid sequence as set out in any one of Figures 4 to 6.

10. The nucleic acid molecule according to claim 8, having greater than 90% nucleic acid sequence identity with any one of the nucleic acid sequences shown in Figures 4 to 6.

11. An expression vector comprising a nucleic acid sequence according to any one of claims 8 to 10 for use in therapy.

12. Use of glycosylphosphatidyl inositol specific phospholipase D (GPI-PLD) according to any one of claims 1 to 7 for the preparation of a medicament for the treatment of diabetes or diabetic complications.

13. The use according to claim 12, wherein the diabetes is an insulin dependent form of diabetes.

14. The use according to claim 12 or claim 13, wherein the diabetes is Type I or Type II diabetes.

15. The use according to claim 14, wherein the complications of diabetes are due to insulin resistance.

16. The use according to any one of claims 12 to 15, wherein the medicament further comprises insulin, a glucose sparing or insulin enhancing drug, an α-glucosidase inhibitor or drug to treat insulin sensitivity, a P and/or A-type inositolphosphoglycan (IPG) and/or an IPG antagonist.

17. Use of a nucleic acid molecule encoding GPI-PLD according to any one of claims 8 to 10 for the preparation of a medicament for the treatment of diabetes or diabetic complications.

18. Use of a glycosylphosphatidyl inositol specific phospholipase D (GPI-PLD) according to any one of claims 1 to 7 for the preparation of a medicament for the treatment of liver dysfunction or disorders involving pancreatectomies.

19. The use according to claim 18, wherein the medicament further comprises apolipoprotein A1.

20. Use of a nucleic acid molecule encoding GPI-PLD according to any one of claims 8 to 10 for the preparation of a medicament for the treatment of liver dysfunction.

21. The use according to claims 20, wherein the liver dysfunction is **characterised by** reduced levels of apolipoprotein A1 and/or GPI PLD and/or apolipoprotein A1/GPI-PLD complex as compared to a normal patient.

22. A cell line transformed with nucleic acid encoding GPI-PLD according to any one of claims 8 to 10, and capable of expressing and secreting GPI-PLD, for use in therapy.

23. The cell line according to claim 22, wherein the cell line is capable of producing apolipoprotein A1.

24. The cell line according to claim 22 or claim 23, wherein the cell line is a yeast cell line, a eukaryotic cell line or a prokaryotic cell line.

25. The use of the cell line according to any one of claims 22 to 24, in the preparation of a medicament for treatment of diabetes or diabetic complications, liver dysfunction or disorders involving pancreatectomies, a condition mediated by a product of an infectious organism which is capable of inhibiting GPI-PLD, or septic shock.

26. A pharmaceutical composition comprising a nucleic acid molecule encoding a GPI-PLD protein according to any one of claims 8 to 10.

27. A pharmaceutical composition comprising a GPI-PLD protein according to any one of claims 1 to 7.

28. The pharmaceutical composition according to claim 27, further comprising (a)P- and/or A-type inositolphosphoglycans, or (b) insulin, an oral insulin composition, a glucose sparing or insulin enhancing drug, an a-glucosidase inhibitor capable of reducing carbohydrate absorption, a drug to treat insulin sensitivity, or(c) apolipoprotein A1.

## Patentansprüche

1. Isolierte, Glykosylphosphatidylinosit-spezifische Phospholipase D (GPI-PLD) zur therapeutischen Verwendung, wobei das GPI-PLD-Polypeptid Folgendes umfasst:
(a) ein GPI-PLD-Polypeptid mit einer Aminosäuresequenz wie in in Fig. 1 oder 3 angeführt; oder
(b) ein GPI-PLD-Polypeptid, das mehr als 70 % Aminosäuresequenzidentität mit der unter (a) definierten Aminosäuresequenz aufweist und die enzymatische Aktivität zum Spalten der Phosphodiesterbindung, die GPI an die Phosphatidsäure bindet, beibehält;
(c) ein GPI-PLD-Polypeptid, das ein aktiver Abschnitt des unter (a) beschriebenen GPI-PLD-Polypeptids ist; oder
(d) ein GPI-PLD-Polypeptid wie unter (a), (b) oder (c) definiert, das an einen Bindungspartner gebunden ist.

2. GPI-PLD nach Anspruch 1, worin die GPI-PLD und Apolipoprotein A1, ein Inositphosphoglykan (IPG) vom P- und/oder A-Typ und/oder ein IPG-Antagonist gleichzeitig oder nacheinander verabreicht werden.

3. GPI-PLD-Polypeptid nach Anspruch 1, das im Vergleich zu einer in Fig. 1 oder 3 angeführten Aminosäuresequenz eine Insertion, Addition, Deletion oder Substitution von 1 bis 10 Aminosäuren aufweist.

4. GPI-PLD nach einem der Ansprüche 1 bis 3, worin der Bindungspartner ein Effektor, eine Markierung, ein Arzneimittel, ein Toxin, ein Träger oder ein Transportmolekül ist.

5. GPI-PLD-Polypeptid nach einem der vorangegangenen Ansprüche, worin die GPI-PLD sich in der Region, die den Aminosäuren 689-692 (Grenzen eingeschlossen) (RRFS) von menschlicher GPI-PLD mit der in Fig. 1 angeführten Aminosäuresequenz entspricht, an einem oder mehreren Resten in der Aminosäuresequenz unterscheidet.

6. GPI-PLD-Polypeptid nach Anspruch 5, das in der Region, die den Aminosäuren 689-692 von menschlicher GPI-PLD mit der in Fig. 1 dargestellten Aminosäuresequenz entspricht, eine Substitution aufweist.

7. GPI-PLD nach Anspruch 6, worin durch die Substitution der Serinrest an Position 692 zu einer anderen Aminosäure als Serin oder Threonin geändert ist.

8. Isoliertes Nucleinsäuremolekül, das für ein GPI-PLD-Polypeptid nach einem der Ansprüche 1 bis 7 kodiert, zur therapeutischen Verwendung.

9. Nucleinsäuremolekül nach Anspruch 8 mit der in einer der Fig. 4 bis 6 angeführten Nucleinsäuresequenz.

10. Nucleinsäuremolekül nach Anspruch 8 mit mehr als 90 % Nucleinsäuresequenzidentität mit einer der in den Fig. 4 bis 6 angeführten Nucleinsäuresequenzen.

11. Expressionsvektor, umfassend eine Nucleinsäuresequenz nach einem der Ansprüche 8 bis 10 zur therapeutischen Verwendung.

12. Verwendung von Glykosylphosphatidylinosit-spezifischer Phospholipase D (GPI-PLD) nach einem der Ansprüche 1 bis 7 zur Herstellung eines Medikaments zur Behandlung von Diabetes und von mit Diabetes zusammenhängenden Komplikationen.

13. Verwendung nach Anspruch 12, worin die Diabeteserkrankung eine insulinabhängige Form von Diabetes ist.

14. Verwendung nach Anspruch 12 oder 13, worin die Diabeteserkrankung Typ-I- oder Typ-II-Diabetes ist.

15. Verwendung nach Anspruch 14, worin die mit Diabetes zusammenhängenden Komplikationen auf Insulinresistenz zurückzuführen sind.

16. Verwendung nach einem der Ansprüche 12 bis 15, worin das Medikament weiters Insulin, ein Glucose senkendes oder Insulin steigerndes Arzneimittel, einen α-Glucosidase-Hemmer oder ein Arzneimittel zur Behandlung von Insulinsensibilität, ein Inositphosphoglykan (IPG) vom P- und/oder A-Typ und/oder einen IPG-Antagonisten umfasst.

17. Verwendung eines für GPI-PLD kodierenden Nucleinsäuremoleküls nach einem der Ansprüche 8 bis 10 zur Herstellung eines Medikaments zur Behandlung von Diabetes oder von mit Diabetes zusammenhängenden Komplikationen.

18. Verwendung von Glykosylphosphatidylinosit-spezifischer Phospholipase D (GPI-PLD) nach einem der Ansprüche 1 bis 7 zur Herstellung eines Medikaments zur Behandlung von Leberdysfunktion und Leberstörungen, die Pankreatektomien bedingen.

19. Verwendung nach Anspruch 18, worin das Medikament weiters Apolipoprotein A1 umfasst.

20. Verwendung eines für GPI-PLD kodierenden Nucleinsäuremoleküls nach einem der Ansprüche 8 bis 10 zur Herstellung eines Medikaments zur Behandlung von Leberdysfunktion.

21. Verwendung nach Anspruch 20, worin die Leberdysfunktion durch im Vergleich zu einem gesunden Patienten niedrigere Spiegel von Apolipoprotein A1 und/oder von GPI-PLD und/oder des Apolipoprotein-Al/GPI-PLD-Komplexes **gekennzeichnet** ist.

22. Mit einer für GPI-PLD kodierenden Nucleinsäure nach einem der Ansprüche 8 bis 10 transformierte Zelllinie, die in der Lage ist, GPI-PLD zu exprimieren und zu sekretieren, zur therapeutischen Verwendung.

23. Zelllinie nach Anspruch 22, worin die Zelllinie in der Lage ist, Apolipoprotein A1 zu produzieren.

24. Zelllinie nach Anspruch 22 oder 23, worin die Zelllinie eine Hefezelllinie, eine eukaryotische Zelllinie oder eine prokaryotische Zelllinie ist.

25. Verwendung der Zelllinie nach einem der Ansprüche 22 bis 24 zur Herstellung eines Medikaments zur Behandlung von Diabetes oder von mit Diabetes zusammenhängenden Komplikationen, Leberdysfunktion oder Leberstörungen, die Pankreatektomien bedingen, einer durch ein Produkt eines infektiösen Organismus, der in der Lage ist, GPI-PLD zu hemmen, vermittelten Erkrankung oder von septischem Schock.

26. Pharmazeutische Zusammensetzung, umfassend ein für ein GPI-PLD-Protein kodierendes Nucleinsäuremolekül nach einem der Ansprüche 8 bis 10.

27. Pharmazeutische Zusammensetzung, umfassend ein GPI-PLD-Protein nach einem der Ansprüche 1 bis 7.

28. Pharmazeutische Zusammensetzung nach Anspruch 27, die weiters Folgendes umfasst: (a) Inositphosphoglykane vom P- und/oder A-Typ oder (b) Insulin, eine orale Insulinzusammensetzung, ein Glucose senkendes oder Insulin steigerndes Arzneimittel, einen A-Glucosidase-Hemmer, der in der Lage ist, die Kohlenhydratabsorption zu hemmen, oder ein Arzneimittel zur Behandlung von Insulinsensibilität oder (c) Apolipoprotein A1.

## Revendications

1. Phospholipase D spécifique au glycosylphosphatidylinositol (GPI-PLD) isolée destinée à un usage thérapeutique, où le polypeptide de GPI-PLD comprend :
(a) un polypeptide de GPI-PLD ayant une séquence d'acides aminés telle que présentée sur la figure 1 ou 3 ; ou
(b) un polypeptide de GPI-PLD ayant une identité de séquence d'acides aminés supérieure à 70 % avec la séquence d'acides aminés définie en (a) qui conserve l'activité enzymatique de clivage de la liaison phosphodiester liant le GPI à l'acide phosphatidique ; ou
(c) un polypeptide de GPI-PLD qui est une partie active du polypeptide de GPI-PLD tel que présenté en (a) ; ou
(d) un polypeptide de GPI-PLD tel que défini en (a), (b), ou (c) qui est lié à un partenaire de couplage.

2. GPI-PLD selon la revendication 1, où la GPI-PLD est simultanément ou séquentiellement administrée avec une apolipoprotéine A1, un inositol-phosphoglycane (IPG) de type P et/ou A et/ou un antagoniste d'un IPG.

3. Polypeptide de GPI-PLD selon la revendication 1, comportant une insertion, addition, délétion ou substitution de 1 à 10 acides aminés par comparaison à une séquence d'acides aminés telle que représentée sur la figure 1 ou 3.

4. GPI-PLD selon l'une quelconque des revendications 1 à 3, où le partenaire de couplage est un effecteur, un marqueur, un médicament, une toxine, un véhicule ou une molécule de transport.

5. Polypeptide de GPI-PLD selon l'une quelconque des revendications précédentes, où la GPI-PLD a une séquence d'acides aminés qui diffère au niveau de un ou plusieurs résidus dans la région correspondant aux acides aminés 689-692 inclus (RRFS) d'une GPI-PLD humaine ayant la séquence d'acides aminés montrée sur la figure 1.

6. Polypeptide de GPI-PLD selon la revendication 5, qui comprend une substitution dans la région correspondant aux acides aminés 689-692 d'une GPI-PLD humaine ayant la séquence d'acides aminés montrée sur la figure 1.

7. GPI-PLD selon la revendication 6, où la substitution modifie le résidu sérine à la position 692 en un acide aminé autre que la sérine ou la thréonine.

8. Molécule d'acide nucléique isolée codant pour un polypeptide de GPI-PLD selon l'une quelconque des revendications 1 à 7 destiné à un usage thérapeutique.

9. Molécule d'acide nucléique selon la revendication 8, ayant la séquence d'acide nucléique telle que représentée sur l'une quelconque des figures 4 à 6.

10. Molécule d'acide nucléique selon la revendication 8, ayant une identité de séquence d'acide nucléique supérieure à 90 % avec l'une quelconque des séquences d'acide nucléique montrées sur les figures 4 à 6.

11. Vecteur d'expression comprenant une séquence d'acide nucléique selon l'une quelconque des revendications 8 à 10 destinée à un usage thérapeutique.

12. Utilisation d'une phospholipase D spécifique au glycosylphosphatidylinositol (GPI-PLD) selon l'une quelconque des revendications 1 à 7, pour la préparation d'un médicament destiné au traitement du diabète ou de complications du diabète.

13. Utilisation selon la revendication 12, où le diabète est une forme de diabète insulinodépendante.

14. Utilisation selon la revendication 12 ou la revendication 13, où le diabète est un diabète de type I ou de type II.

15. Utilisation selon la revendication 14, où les complications du diabète sont dues à une résistance à l'insuline.

16. Utilisation selon l'une quelconque des revendications 12 à 15, où le médicament comprend en outre de l'insuline, un médicament épargnant le glucose ou stimulant l'insuline, un inhibiteur de l'α-glucosidase ou un médicament pour traiter une sensibilité à l'insuline, un inositol-phosphoglycane (IPG) de type P et/ou de type A et/ou un antagoniste d'un IPG.

17. Utilisation d'une molécule d'acide nucléique codant pour une GPI-PLD selon l'une quelconque des revendications 8 à 10, pour la préparation d'un médicament destiné au traitement du diabète ou de complications du diabète.

18. Utilisation d'une phospholipase D spécifique au glycosylphosphatidylinositol (GPI-PLD) selon l'une quelconque des revendications 1 à 7, pour la préparation d'un médicament destiné au traitement d'un dysfonctionnement hépatique ou de troubles impliquant des pancréatectomies.

19. Utilisation selon la revendication 18, où le médicament comprend en outre une apolipoprotéine A1.

20. Utilisation d'une molécule d'acide nucléique codant pour une GPI-PLD selon l'une quelconque des revendications 8 à 10, pour la préparation d'un médicament destiné au traitement d'un dysfonctionnement hépatique.

21. Utilisation selon la revendication 20, où le dysfonctionnement hépatique est **caractérisé par** des taux réduits d'apolipoprotéine A1 et/ou de GPI-PLD et/ou de complexe apolipoprotéine A1/GPI-PLD par comparaison à un patient normal.

22. Lignée cellulaire transformée avec un acide nucléique codant pour une GPI-PLD selon l'une quelconque des revendications 8 à 10, et capable d'exprimer et de sécréter une GPI-PLD destinée à un usage thérapeutique.

23. Lignée cellulaire selon la revendication 22, où la lignée cellulaire est capable de produire une apolipoprotéine A1.

24. Lignée cellulaire selon la revendication 22 ou la revendication 23, où la lignée cellulaire est une lignée cellulaire de levure, une lignée cellulaire eucaryote ou une lignée cellulaire procaryote.

25. Utilisation de la lignée cellulaire selon l'une quelconque des revendications 22 à 24, dans la préparation d'un médicament destiné au traitement du diabète ou de complications du diabète, d'un dysfonctionnement hépatique ou de troubles impliquant des pancréatectomies, d'une pathologie médiée par un produit d'un organisme infectieux qui est capable d'inhiber une GPI-PLD, ou un choc septique.

26. Composition pharmaceutique comprenant une molécule d'acide nucléique codant pour une protéine GPI-PLD selon l'une quelconque des revendications 8 à 10.

27. Composition pharmaceutique comprenant une protéine GPI-PLD selon l'une quelconque des revendications 1 à 7.

28. Composition pharmaceutique selon la revendication 27, comprenant en outre (a) des inositol-phosphoglycanes de type P et/ou de type A, ou (b) de l'insuline, une composition d'insuline orale, un médicament épargnant le glucose ou stimulant l'insuline, un inhibiteur de l'a-glucosidase capable de réduire l'absorption des hydrates de carbone, un médicament pour traiter une sensibilité à l'insuline, ou (c) une apolipoprotéine A1.
